# EUROPÄISCHE PATENTSCHRIFT

(11) **EP 1 420 749 B1**
(45) Veröffentlichungstag und Bekanntmachung des Hinweises auf die Patenterteilung: **17.10.2007**
(21) Anmeldenummer: 02767127.0
(22) Anmeldetag: 28.08.2002
(51) Int. Cl.: A61K 8/04, A61K 8/34, A61K 8/36, A61K 8/365, A61K 8/37, A61K 8/49, A61K 8/60, A61Q 5/02, A61Q 15/00, A61Q 19/10

(54) **SPRÜHBARE O/W-EMULSIONEN VON NIEDRIGER VISKOSITÄT**
SPRAYABLE O/W EMULSIONS OF A LOW VISCOSITY
EMULSIONS HUILE DANS EAU PULVERISABLES PRESENTANT UNE FAIBLE VISCOSITE

(30) Priorität: 28.08.2001 DE 10141324
(43) Veröffentlichungstag der Anmeldung: 26.05.2004
(73) Patentinhaber: SASOL Germany GmbH, 20537 Hamburg (DE)
(72) Erfinder: KWETKAT, Klaus, D-59192 Bergkamen (DE); DAHMS, Gerd, M., 47138 Duisburg (DE)
(74) Vertreter: Schupfner, Georg
(86) Internationale Anmeldenummer: PCT/DE2002/003180
(87) Internationale Veröffentlichungsnummer: WO 2003/024412

(56) Entgegenhaltungen:
- DE-A- 19 637 840
- G.H. DAHMS: "Stabile, flüssige Öl-in-Wasser Emulsionen auf Basis von Gemini-Surfactants" SÖFW-JOURNAL, Bd. 126, Nr. 9, 2000, Seite 34-36,38,39 XP000981707 DE
- K. KWETKAT: "Gemini surfactants- Applications in real life" WORLD SURFACTANTS CONGRESS, 5TH, FIRENZE, ITALY, MAY 29-JUNE 2, 2000. PUBLISHER: COMIT¹ EUROP¹EN DES AGENTS DE SURFACE ET LEURS INTERM¹DIAIRES ORGANIQUES, BRUSSELS, BE, Seiten 1107-1118, XP008016234

## Beschreibung

Die vorliegende Erfindung betrifft sprühbare O/W-Emulsionen von niedriger Viskosität.

Sprühbar im Sinne der Erfindung heißt, dass die O/W-Emulsionen insbesondere mittels einer sogenannten Fingerpumpe oder Triggerpumpe direkt als O/W-Emulsion ohne die Hilfe eines unter Druck stehenden Treibmittels versprühbar sind.

Aus dem Stand der Technik sind sprühbare O/W-Emulsionen, die entweder auf Basis von nichtionischen Tensiden mit Hilfe der Phaseninversionsmethode hergestellt werden oder auf anionischen Emulgatoren, wie z.B. Glyceryl Stearat Citrat oder Phosphorsäurederivate und einem Verdickungssystem beruhen, bekannt.

Besonders als Sonnenschutzprodukte erfreuen sich die sprühbaren O/W-Emulsionen wachsender Popularität beim Verbraucher. Die aus dem Stand der Technik bekannten sprühbaren O/W-Emulsionen, haben den Nachteil, dass bisher keine Zusammensetzungen bekannt sind, die akzeptable Konzentrationen der in Cremes oder Lotionen bewährten UV-Filter Kombinationen aus organischen öl- oder wasserlöslichen UV-Filtern und anorganischen UV-Filtern, wie Titandioxid, das amphiphil, lipophil oder hydrophob beschichtet sein kann, und / oder Zinkoxid, erlauben. Weiterhin ist es nicht möglich, höhere Ethanolkonzentrationen einzusetzen und somit die Formulierung konservierungsmittelfreier Produkte zu ermöglichen.

Um einen besonders feinen Spraynebel und Sprayfilm mit konventionell verfügbaren Pumpsprühvorrichtungen zu erzielen, ist eine niedrige Viskosität der zu versprühenden Zusammensetzungen Voraussetzung. Gleichzeitig muss die Handhabung der Handpumpe einfach sein und eine für kosmetische Anwendungen ansprechende Gestaltung der Produktverpackung ermöglichen. Die Handpumpe soll unter gleichmäßiger und leichter Kraftanwendung durch den Benutzer im Wesentlichen unabhängig von der Pumpgeschwindigkeit einen feinen und gleichmäßigen Sprühnebel bilden. Hochviskose Zusammensetzungen machen stabile großvolumige und unansehnliche Handpumpen erforderlich und führen meist zu schlechten Sprühergebnissen.

Tensidmischungen zur Herstellung sprühbarer O/W Emulsionen mit geringer Viskosität enthaltend neben Geminitensiden weitere Tenside sind an sich bekannt, z.B. aus G.H. Dahms "Stabile, flüssige Öl-in-Wasser Emulsionen auf der Basis von Geminitensiden" in SÖFW-Journal, Bd. 125, Nr. 9, 2000, Seite 34-36, 38-39. In dieser Veröffentlichung wird aber weder eine Einarbeitung weiterer Bestandteile wie Feststoffpartikel, schäumendes Tensid oder Antitranspirant in die sprühbare O/W Emulsionen offenbart noch der geforderte Wassergehalt oder das Verhältnis von schwach-schäumenden Tensid zu Geminitensid.

Aufgrund der im Vergleich zur Wasserphase hohen Dichte anorganischer Lichtschutzfilter neigen O/W-Emulsionen mit niedriger Viskosität mit zunehmender Pigmentkonzentration zu erheblichen Instabilitäten, d.h. die suspendierten Festkörper neigen zur Agglomeration und/oder sedimentieren.

Nach dem Stand der Technik hat man versucht thixotrope pigmenthaltige Zusammensetzungen herzustellen, die unter dem Einfluss von Scherkräften versprühbar sind. Aber auch hierdurch werden versprühbare Zusammensetzungen mit hohen Festkörperpartikelkonzentrationen nicht erhältlich.

Festkörperpartikel-Konzentrationen von 4 % oder größer, wie sie in Sonnenschutz-Cremes oder -Lotionen üblich sind, stellen herkömmlich ein für die Anwendung "sprühbare Zusammensetzung" unüberwindbares Problem dar. Höhere Konzentrationen an Festkörperpartikeln sind in den vorgenannten Zusammensetzungen nach dem Stand der Technik kaum zu realisieren, insbesondere auch deshalb, da aufgrund der Agglomerationsneigung der anorganischen UV-Filter-Partikel, insbesondere nach dem Aufbringen auf die Haut, diese als weiße Schicht sichtbar werden, die als sogenanntes "Weißeln" bezeichnet wird.

Der Verbraucher wiederum findet sogenannte "Pump-Sprays", die nicht einen Aerosol-Sprays ähnlichen feinen Sprühnebel ergeben, sondern mit einem breiten Strahl auf die Haut auftreffen, als sehr unattraktiv.

Für den Hersteller von Verbraucherprodukten wiederum ist es wünschenswert, das Emulgator /Dispergator - System so wenig wie möglich an verschiedene Feststoffpartikel, seien es nun anorganische UV-Filter oder Effektpigmente für die eher dekorative Kosmetik oder Füllstoffe für technische Anwendungen, anzupassen.

Aufgabe der Erfindung war es daher eine niedrigviskose, sprühbare O/W-Emulsion mit Hilfe einer Emulgator- und Dispergator-Zusammensetzung bereitzustellen, die flexibel und einfach für eine Vielzahl unterschiedlicher Feststoffpartikel und Feststoffpartikel-Konzentrationen einsetzbar ist und die oben geschilderten Nachteile des Standes der Technik nicht aufweist.

Diese Aufgabe wird überraschend gelöst von nachfolgender Zusammensetzung:

Öl in Wasser Emulsion (O/W=Emulsion) mit einer Viskosität von kleiner 2000 mPas, vorzugsweise 50 bis 1.000 mPas, besonders bevorzugt 500 bis 1000 mPas (bei Raumtemperatur 298K , gemessen als dynamische Viskosität bei einer Scherung von 1/s) herstellbar unter Zusammenbringen
(a) einer *hydrophilen Phase* enthaltend:
   (a.1) eines oder mehrere Geminitensid-Verbindungen und
   (a.2) eine Detergenskomponente mit schlecht schäumender Charakteristik, im Gewichtsverhältnis Geminitensid-Verbindung (a.1) zu Detergenskomponente (a.2) von 1 zu 100 bis 10 zu 1, vorzugsweise 1 zu 10 bis 4 zu 1, besonders bevorzugt 1 zu 2 bis 2 zu 1, Gewichtsteilen, wobei die Komponenten (a.1) und (a.2) als Summe von (a.1) und (a.2) vorzugsweise zu 0,05 bis 5,0 Gew.%, besonders bevorzugt 0,5 bis 3,0 Gew.%, eingesetzt werden,
   (a.3) 1 bis 15 Gew.% Wasser, besonders bevorzugt 2 bis 10 Gew.%,
(b) mit einer *hydrophoben* Phase enthaltend:
   (b.1) eine oder mehrere Geminitensid-Verbindungen und
   (b.2) ein oder mehrere Co-Amphiphile mit einem HLB- Wert von kleiner 6, im Gewichtsverhältnis Geminitensid-Verbindung (b.1) zu Co-Amphiphil (b.2) von 1 zu 100 bis 3 zu 1, vorzugsweise 1 zu 20 bis 1 zu 2, besonders bevorzugt 1 zu 10 bis 1 zu 5, Gewichtsteilen,
      wobei die Komponenten (b1) und (b2) als Summe von (b.1) und (b.2) vorzugsweise zu 0,1 bis 8,0 Gew.%, besonders bevorzugt von 0,5 bis 4,0 Gew.%, eingesetzt werden,
   (b.3) 1 bis 50 Gew.%, vorzugsweise 10 bis 40 Gew.%, einer hydrophoben Komponente und,
   (b.4) ggf. 0,01 bis 10 Gew.%, vorzugsweise 0,5 bis 3 Gew.% eines oder mehrerer nichtionischer Tenside ;
   vorzugsweise bei erhöhter Temperatur, besonders bevorzugt 50 bis 80°C bzw. 60 bis 70°C, und vorzugsweise unter Homogenisieren bis zu einer mittleren Teilchengröße der Öltropfchen gemäß Streulichtbestimmung von < 1 mm, vorzugsweise von < 10 µm, und weiterhin enthaltend:
   (c.1) Wasser, so dass eine Zusammensetzung enthaltend 15 Gew.% bis 45 Gew.% Wasser resultiert, vorzugsweise durch Zugabe zur Phase (a) oder nach dem Zusammenbringen der Phasen (a) und (b),
   (c.2) Alkohole (c.2.1), Polyglykole (c.2.2) und/oder Polyole (c.2.3), so dass eine Gesamtzusammensetzung enthaltend in der Summe 0,1 bis 50 Gew.%, vorzugsweise 5 bis 30 Gew.% Alkohole (c.2.1), Polyglykole (c.2.2) und/oder Polyole (c.2.3) resultiert,
      vorzugsweise durch Zugabe zur Phase (a) und/oder (b) und/oder nach dem Zusammenbringen der Phasen (a) und (b),
      wobei der Phase (a) vorzugsweise 0,1 bis 10 Gew.% Polyole (c.2.3) zugegeben sind,
   (c.4) ggf. einen Viskositätsregulator, vorzugsweise 0,01 bis 3 Gew.%, vorzugsweise 0,2 bis 1 Gew.%, vorzugsweise durch Zugabe zur Phase (a) oder nach dem Zusammenbringen der Phasen (a) und (b),
   (c.5) ggf. vernetzte Polymere, vorzugsweise 0,1 bis 1 Gew.%, vorzugsweise durch Zugabe nach dem Zusammenbringen der Phasen (a) und (b),
   und eine oder mehrere der Komponenten (d.1) bis (d.3) durch Zugabe nach dem Zusammenbringen der Phasen (a) und (b), ggf. gemeinsam mit einer der Komponenten
(c) ((c.1) bis (c.5)), von:
   (d.1) 0,1 bis 30 Gew.%, vorzugsweise 0,1 % bis 30 Gew.%, Feststoffpartikeln,
   (d.2) 0,1 bis 3 Gew.%, vorzugsweise 0,5 % bis 2 Gew.%, eines schäumenden Tensids, vorzugsweise stark schäumenden Tensids oder
   (d.3) 0,1 bis 15 Gew.%, vorzugsweise 2 bis 8 Gew.%, eines Antitranspirants, wie eines Metall-Chloro-Hydrats, insbesondere Aluminium-Chloro-Hydrats oder Zirkonium-Chloro-Hydrats,
   wobei im Falle der Zugabe des schäumenden Tensids (d.2), eine Zugabe von zusätzlichem Wasser (zusätzlich zu (c.1)) erfolgt, so dass sich ein Verhältnis von Öl in Wasser Emulsion (Komponenten (a) bis (c)) zu zusätzlichem Wasser einschließlich schäumenden Tensid von 90:10 bis 40 : 60 Gewichtsteilen ergibt und wobei die Geminitenside mindestens 2 Tensideinheiten umfassen, die jeweils mindestens eine hydrophile Kopfgruppe und mindestens eine hydrophobe Gruppe aufweisen, und die Tensideinheiten durch mindestens einen Spacer in der Nähe der Kopfgruppe verknüpft sind.

Vorzugsweise erfolgt die Zugabe von zusätzlichem Wasser gemeinsam mit dem schäumenden Tensid (d.2) oder vor Zugabe des schäumenden Tensids. Nach Zugabe der oben genannte Komponenten kann eine weitere Zugabe von Wasser zur O/W-Emulsion auch über oben genannte Konzentrationen hinaus erfolgen.

Die Öl in Wasser Emulsion enthält vorzugsweise ggf. weiterhin eine oder mehrere der folgenden Komponenten:
- 0,1 bis 20 Gew.%, vorzugsweise 1 bis 15 Gew.%, vorzugsweise 1 bis 8 Gew.%, Ethanol als oder als Teil der Komponente (c.2.1),
   (d.4) 0,1 % bis 25 Gew.% eines organischen löslichen Lichtschutzfilters, vorzugsweise durch Zugabe zur Phase (b) oder, 0,1 bis 25 Gew.% eines wasserlöslichen Lichtschutzfilters oder seines Salzes durch Zugabe zum nach dem Zusammenbringen der Phasen (a) und (b) zugesetzten Wasser.
   (d.2) und (d.3) werden vorzugsweise in wässriger Lösung oder Dispersion zugesetzt.

Vorstehende Gew.% Angaben beziehen sich jeweils auf die Gesamtzusammensetzung O/W-Emulsion. Vorteilhafte Ausführungsformen der Erfindung sind Gegenstand der Unteransprüche.

Eine kommerzielle Ausführungsform enthaltend die Komponenten (a.1) und (a.2) im geeigneten Verhältnis ist das Produkt CERALUTION® F der Sasol Germany GmbH. Eine Zusammensetzung enthaltend die Komponenten (a.1) und (a.2) ist Gegenstand der WO 01/19943-A1 (PCT/DE 00/03163), die hiermit durch Bezugnahme ausdrücklich auch zum Inhalt dieses Schutzrechtes gemacht wird.

Eine besonderes geeignete Ausführungsform enthaltend die Komponenten (a.1) und (a.2) besteht aus Natrium Lauroyl Lactylat und Geminitensid im Verhältnis 1:2 bis 2:1 Gewichtsteilen.

Eine kommerzielle Ausführungsform enthaltend die Komponenten (b.1) und (b.2) im geeigneten Verhältnis ist das Produkt CERALUTION® H der Sasol Germany GmbH. Eine Zusammensetzung enthaltend die Komponenten (b.1) und (b.2) ist Gegenstand der WO 01/19945-A1 (PCT/DE00/03162), die hiermit durch Bezugnahme ausdrücklich auch zum Inhalt dieses Schutzrechtes gemacht wird.

Eine besonderes geeignete Ausführungsform besteht bezüglich der Komponenten (b.1) und (b.2) aus: (1) 10 bis 50 Gew.% Behenyl Alkohol, (2) 30 bis 50 Gew.% Glyceryl Stearat, (3) 10 bis 20 Gew.% Glyceryl Stearat Citrat und (4) 10 bis 20 Gew.% Geminitensid (vorzugsweise Typ A I, mit 15 EO, Ethylen als R² und mit Acylresten aus einem Kokosschnitt (C12, C14 und wenig C16, die polare Endgruppe ist -SO₃Na).

Die Geminitenside (a.1) und (b.1) können gleich oder verschieden sein.

Die erfindungsgemäßen O/W-Emulsionen zeichnen sich dadurch aus, dass > 90 %, bevorzugt > 95 % und besonders bevorzugt > 98 % der Öltröpfchen laut Partikelgrößenbestimmung mit einer Streulichtmethode einen Durchmesser von vorzugsweise < 1 mm, besonders bevorzugt < 10 µm, aufweisen (zumindest nach Homogenisieren der Phasen (a) und (b)). Die Bestimmung der Partikelgrößen erfolgte mit dem Particle Size Analyser HORIBA LA-500.

Die hydrophobe Phase (b) bezeichnet eine Zusammensetzung die bei Raumtemperatur nicht in Wasser löslich ist, ggf. aber in Wasser dispergierbar ist. Die hydrophile Phase (a) ist relativ zur jeweiligen hydrophoben Phase (b) definiert. Vorzugsweise ist die hydrophile Phase in Wasser bei Raumtemperatur ggf, nach vorherigen Erhitzen (nicht dauerhaft) löslich.

Die überlegene Sprühcharakteristik der erfindungsgemäßen O/W-Emulsionen lässt sich durch nachfolgenden Versuch quantifizieren: Im Abstand von 27 cm von einem Sprühpumpenkopf, der einen runden Sprühkegel hat, wird ein Blatt Papier befestigt, das besprüht wird. Die Größe der besprühten Fläche wird ermittelt. Je größer die Fläche bei Einsatz der gleichen Handpumpe ist, desto besser ist die Versprühbarkeit der Zusammensetzung. Die erfindungsgemäßen Zusammensetzungen (abgesehen von der Schaumanwendung unter Zusatz der Komponente (d.2) zeigen ein ausgezeichnetes Sprühverhalten.

Je kleiner und weniger dispers die Öltröpfchen, desto günstiger die gleichmäßige Verteilung der Pigmente. Bei Verwendung der erfindungsgemäßen O/W-Emulsionen ist es möglich die Pigmente feinteiliger und gleichmäßiger zu verteilen. Hierdurch kann eine signifikant bessere Deckwirkung des Pigmentes erzielt werden. Dies ist für kosmetische Anwendungen beispielsweise Sonnenschutzmitteln von Vorteil, da die Pigmente effizienter genutzt werden können und z.B. ein deutlich höherer Lichtschutzfaktor pro Masseneinheit eingesetzter Feststoffpartikel erreichbar ist. Auch in technischen Anwendungen werden Vorteil erzielt, etwa wenn die Feststoffpartikel ohne Agglomerationsneigung in der Größe Ihrer Primärteilchen dauerhaft dispergiert sein sollen. Dies kann etwa in Lacksystemen von Vorteil sein.

Die Anpassung an stärker hydrophile Festkörperpartikel kann durch Erhöhung des Anteils an (a.1) plus (a.2) und ggf. (b.4) in den oben angegebenen Grenzen erfolgen.

### (a.1) und (b.1) Geminitensid-Verbindungen

Die Geminitenside können als im wesentlichen einheitliche oder als Mischungen unterschiedlicher Verbindungen vorliegen

Unter Geminitensiden versteht man - im Rahmen der vorliegenden Erfindung - grenzflächenaktive Verbindungen, die aus mindestens zwei, vorzugsweise aus zwei, Tensideinheiten, d.h. aus hydrophiler Kopfgruppe und hydrophober Gruppe, bestehen, die durch mindestens einen, vorzugsweise durch einen, Abstandhalter, Spacer genannt, in der Nähe der Kopfgruppe miteinander verknüpft sind. Die Gemini- oder Zwillingstenside werden auch Dimertenside genannt und tragen ihren Namen aufgrund ihrer besonderen Bauart. Abhängig von der Natur der Kopfgruppe gibt es anionische, nichtionische, kationische und amphotere Geminitenside. Anders als konventionelle Tenside, die man ebenfalls in diese Gruppen einteilt, können Geminitenside jedoch auch Kombinationen von Kopfgruppen unterschiedlichen Charakters tragen. Dabei handelt es sich meist um Kombinationen aus nichtionischen und ionischen Gruppen.

Bei Kombinationen von ionischen mit nichtionischen Kopfgruppen überwiegt die Natur der ionischen Kopfgruppe im resultierenden Geminitensid, so daß Kombinationen aus nichtionischer und anionischer Kopfgruppe als anionische Geminitenside klassifiziert werden. Analoges gilt für die Kombinationen nichtionischer mit kationischen oder amphoteren Kopfgruppen. Für die erfindungsgemäßen Tensidzusammensetzungen ist in erster Linie die Morphologie, also die relative Anordnung der verschiedenen Struktureinheiten (hydrophile Gruppen, Spacer, hydrophobe Ketten) zueinander, entscheidend und nicht die Art der Kopfgruppe. Die erfindungsgemäß eingesetzten Geminitenside weisen demnach folgende Struktur auf:

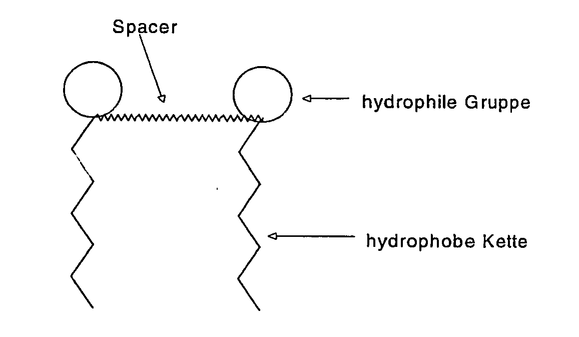

Zu den bevorzugt in den erfindungsgemäßen Tensidzusammensetzungen eingesetzten Geminitensiden zählen solche, die an der Verknüpfungsstelle zwischen Spacer, hydrophiler und hydrophober Gruppe Stickstoffatome enthalten. Dazu gehören bevorzugt Geminitenside mit Amin- oder Amidgruppen aufweisenden Spacern, aber auch sich von Dicarbonsäuren ableitende Spacer, sich von Betainstrukturen ableitende, hydrophile Doppelkopfgruppen, die gegebenenfalls durch Alkoxylieren (insbesondere Ethoxylieren) hergestellte Seitengruppen aufweisen, die Sulfonsäure-, Phosphonsäure-, Carbonsäure oder Alkoholgruppen (einschließlich Polyalkoholen) tragen können, und hydrophobe Doppelketten mit 5 bis 25 C-Atomen, die verzweigt oder unverzweigt sein können und bis zu 2 nicht benachbarte Doppelbindungen tragen können.

Die folgenden Strukturvarianten der Geminitenside sind für die erfindungsgemäße Tensidzusammensetzungen besonders geeignet.

### Variante A: Auf amid- oder aminhaltigen Spacern beruhende Strukturen

A.I Geminitenside der allgemeinen Formel (A.I) analog WO 96/14926 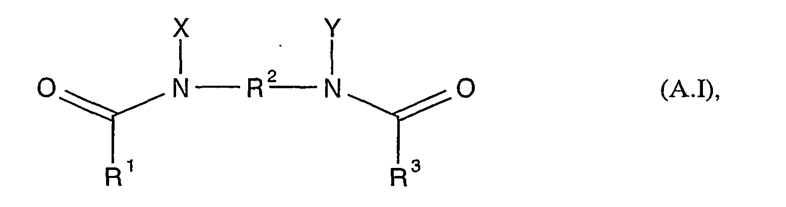 wobei die Substituenten unabhängig voneinander folgende Bedeutung haben:
   - R¹, R³: C₅- bis C₂₅-Alkyl, verzweigt oder unverzweigt, gesättigt, gegebenenfalls bis zu 2-fach nicht-benachbart ungesättigt;
   - R²: C₁- bis C₁₂-Alkylen;
   - X,Y: (C₂H₄O-)ₓ(C₃H₆O-)_{y}-FR; x+y≥1, x: 0-15, y: 0-10 und
   - FR: -SO₃M, -CH₂-CO₂M, -P(O)(OM)₂, H, -C₃H₆SO₃M; oder -CH₂(CHOH)₄CH₂OH, soweit x+y=0; wobei M = ein Gegenion wie Alkali, (Alkyl)Ammonium, Alkanolammonium, H oder ½ Erdalkali ist.
A.II Geminitenside mit Dicarbonsäure-stämmigen Spacern der allgemeinen Formel (A.II) analog WO 96/25388 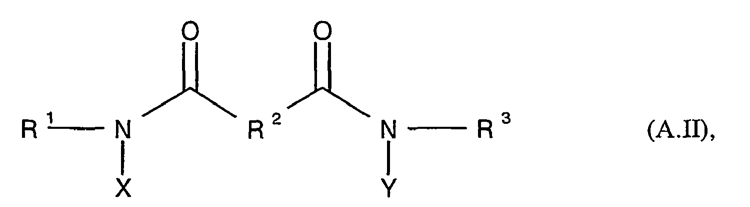 wobei die Substituenten unabhängig voneinander die für die allgemeine Formel (A.I) angegebene Bedeutung haben.
A.III Amphotere Geminitenside der allgemeinen Formel (A.III) analog WO 97/31890 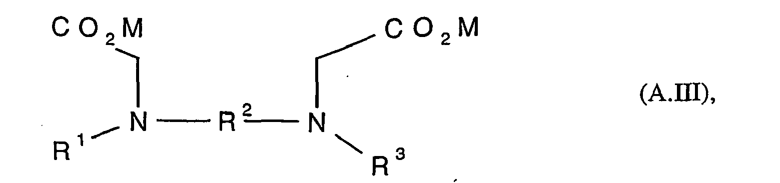 wobei die Substituenten unabhängig voneinander die für die allgemeine Formel (A.I) angegebene Bedeutung haben. Geminitenside der allgemeinen Formel (A.III) sind amphotere Verbindungen, so daß sie bei entsprechend saurem Umgebungsmedium auch kationisch werden können.

### Variante B: Auf amid- oder aminhaltigen Spacern beruhende Strukturen

B.I Geminitenside der allgemeinen Formel (B.I) analog DE 19622612 oder JP-A 10-175934 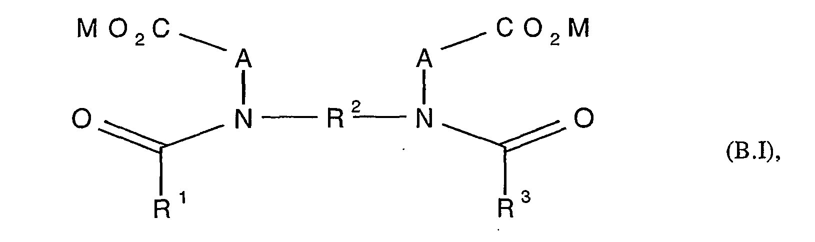 wobei die Substituenten unabhängig voneinander folgende Bedeutung haben:
   - R¹, R³: C₅- bis C₂₅- Alkyl, verzweigt oder unverzweigt, gesättigt, gegebenenfalls bis zu 2-fach nicht-benachbart ungesättigt;
   - R²: C₁- bis C₁₂-Alkylen;
   - A: CHR⁴, CH₂, C₂H₄, C₃H₆, C₄H₈;
   - R⁴: Rest einer Aminocarbonsäure und
   - M: ein Gegenion wie Alkali, (Alkyl)Ammonium, Alkanolammonium, H oder ½ Erdalkali.
B.II Geminitenside der allgemeinen Formel (B.II) analog EP 0 708 079 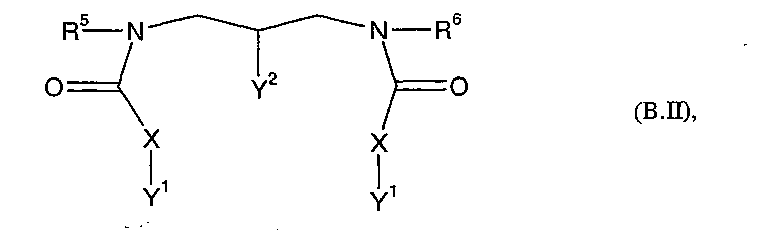 wobei die Substituenten unabhängig voneinander die für die allgemeine Formel (B.1) angegebene Bedeutung haben und
   - R⁵, R⁶: C6- bis C36-Alkyl, verzweigt oder unverzweigt, gesättigt, gegebenenfalls bis zu 2-fach nicht-benachbart ungesättigt;
   - X: Alkylen- oder Alkenylengruppe mit 1 bis 6 Kohlenstoffatomen, die mit einer Hydroxylgruppe oder einer Sulfonsäuregruppe oder einer Carboxygruppe substituiert sein kann;
   - Y¹: eine Sulfonat- oder Sulfatgruppe oder eine Carboxylgrupppe und
   - Y²: eine Hydroxylgruppe, ein Schwefelsäurerest oder -O-(CO)X-COOH bedeuten.
B.III Geminitenside der allgemeinen Formel (B.III) analog JP-A-8-311003 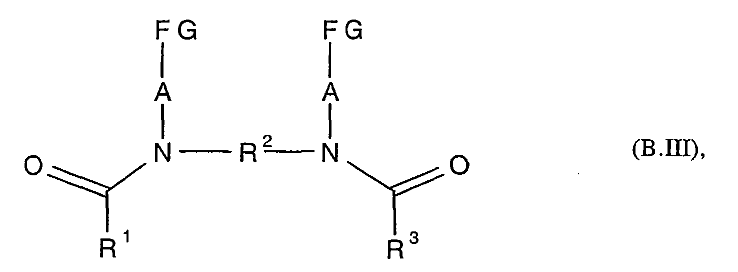 wobei die Substituenten unabhängig voneinander die für die allgemeine Formel (B.I) angegebene Bedeutung haben und
   FG -COOM oder -SO₃M bedeutet.
B.IV Geminitenside der allgemeinen Formel (B.IV) analog JP-A 11-60437 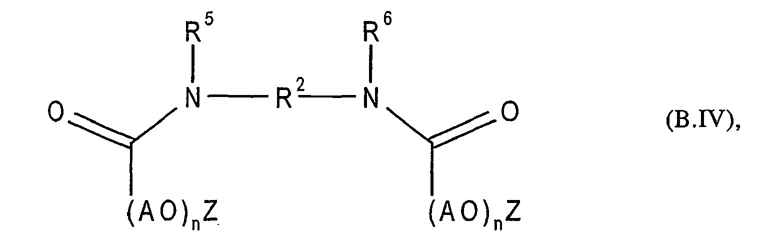 wobei die Substituenten unabhängig voneinander, die bei den allgemeinen Formeln (B.I) und (B.II) angegebene Bedeutung haben und
   - AO: Alkylenoxideinheiten, d.h. Ethylenglykol-, Propylenglykol und Butylenglykolethereinheiten, allein oder statistisch oder blockweise verteilt, mit n = 1 bis 20 und
   - Z: -SO₃M, -C₂H₄SO₃M, -C₃H₆SO₃M, -P(O)(OM)₂ oder -CH₂-COOM, -C₂H₄-COOM bedeuten.
B.V Geminitenside der allgemeinen Formel (B.V) 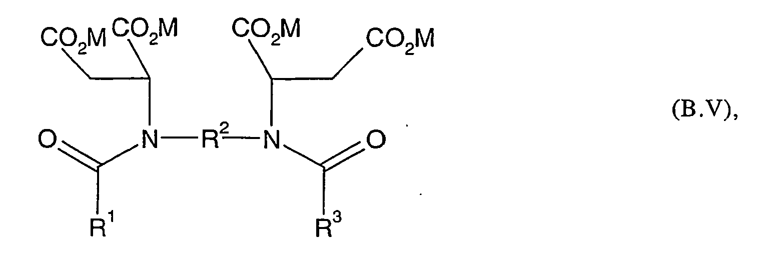 wobei die Substituenten unabhängig voneinander folgende Bedeutung haben:
   - R¹, R³: C₅- bis C₂₅- Alkyl, verzweigt oder unverzweigt, gesättigt, gegebenenfalls bis zu 2-fach nicht-benachbart ungesättigt;
   - R²: C₁- bis C₁₂-Alkylen;
   - M: ein Gegenion wie Alkali, (Alkyl)Ammonium, Alkanolammonium, H oder ½ Erdalkali, wobei die Carbonsäuregruppen auch nur teilweise neutralisiert sein können.

### Variante C: Auf amid- oder aminhaltigen Spacern beruhende Strukturen

C.I Geminitenside der allgemeinen Formel (C.I) analog EP 0 697 244 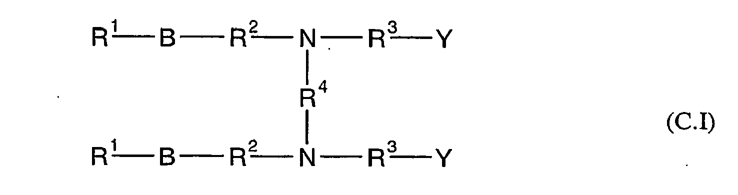 wobei die Substituenten unabhängig voneinander folgende Bedeutung haben:
   - R¹: C₅- bis C₂₅-Alkyl, verzweigt oder unverzweigt, gesättigt, gegebenenfalls bis zu 2-fach nicht-benachbart ungesättigt, hydroxysubstituiert oder perfluoriert;
   - R²: C₁- bis C₁₂-Alkylen oder hydroxysubstituierte Derivate davon;
   - B: eine Amidgruppe [-C(O)N(R²)- oder -N(R⁵)C(O)-], eine Carboxylgruppe [-C(O)O- oder -OC(O)-], eine Polyethergruppe [-(O(R⁶-O)ₓ-];
   - R⁵: für C₁- bis C₄-Alkyl oder hydroxysubstituiertes Alkyl oder H steht;
   - R⁶: für C₂- bis C₄-Alkylen;
   - x: eine Zahl von 1 bis 20;
   - R³: für C₁- bis C₁₂- Alkyl oder hydroxysubstituierte Derivate davon, R⁷-D-R⁷ oder eine Polyethergruppe [-(O(R⁶-O)ₓ-];
   - R⁷: für C₁- bis C₆- Alkylen oder hydroxysubstituierte Derivate davon;
   - D: -O-, -S-, -N(R⁸)- ;
   - R⁴: Alkylen oder Alkylaryl mit 1 bis 12 C-Atomen oder die hydroxysubstituierten Derivaten oder R⁹-D¹-R⁹ ;
   - R⁸: C₁- bis C₁₂-Alkyl oder hydroxysubstituiertes Alkyl oder H oder R⁹-D¹-R⁹;
   - R⁹: für C₁- bis C₆- Alkylen oder hydroxysubstituierte Derivate davon oder Aryl;
   - D¹: -O-, .-S-, -SO₂-, -C(O)-, [-(O(R⁷-O)ₓ-], (R¹⁰)ₜ[N(R¹⁰)]_{z} oder Aryl;
   - R¹⁰: C₁- bis C₁₂-Alkyl oder hydroxysubstituiertes Alkyl oder H oder Aryl;
   - t,z: unabhängig voneinander eine Zahl von 1 bis 4 bedeuten und
   - Y: unabhängig voneinander für -SO₃H, O-SO₃H, -OP(O)(OH)₂, -P(O)(OH)₂, -COOH, -CO₂-C₆H₄-SO₃H und deren Salze davon steht.
C.II Geminitenside der allgemeinen Formel (C.II) analog EP 0 697 245 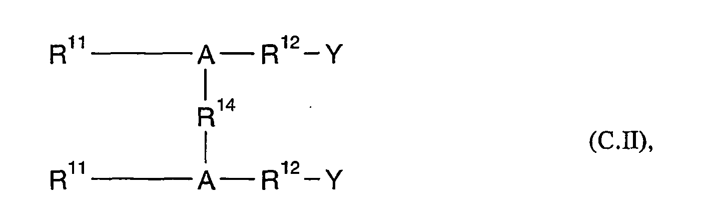 wobei die Substituenten unabhängig voneinander die für die allgemeine Formel (C.I) angegebene Bedeutung haben und
   - R¹¹: C₅- bis C₂₃-Alkyl, verzweigt oder unverzweigt, gesättigt, gegebenenfalls bis zu 2-fach nicht-benachbart ungesättigt, hydroxysubstituiert oder perfluoriert oder R¹⁴-B-R²;
   - R¹⁴: C₁- bis C₁₂-Alkylen, verzweigt oder unverzweigt, gesättigt, gegebenenfalls bis zu 2-fach nicht-benachbart ungesättigt, oder die hydroxysubstituierten Derivate;
   - R¹²: C₁- bis C₁₂-Alkylen, verzweigt oder unverzweigt, gesättigt, gegebenenfalls bis zu 2-fach nicht-benachbart ungesättigt, oder die hydroxysubstituierten Derivate oder eine Amidgruppe [-C(O)N(R²)- oder -N(R⁵)C(O)-], eine Carboxylgruppe [-C(O)O- oder -OC(O)-], eine Polyethergruppe [-(O(R⁶-O)ₓ-] oder R⁹-D¹-R⁹ und
   - A: -CR⁶= oder -N= unter der Voraussetzung, daß wenn A gleich -N= ist, R¹¹ gleich R¹⁴-B-R² bedeuten.
C.III Geminitenside der allgemeinen Formel (C.III) analog DE 4227391 und DE 19608117 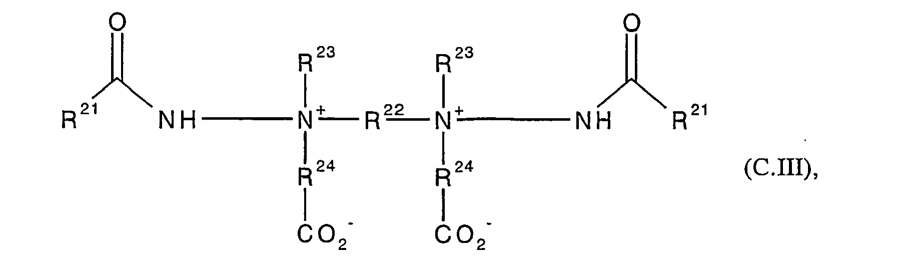 wobei die Substituenten unabhängig voneinander die für die allgemeinen Formeln (C.I) und (C.II) angegebene Bedeutung haben und
   - R²¹: C₅- bis C₂₃-Alkyl, verzweigt oder unverzweigt, gesättigt, gegebenenfalls bis zu 2-fach nicht-benachbart ungesättigt;
   - R²² R²⁴: C₁- bis C₆ Alkylen;
   - R²³: Methyl, Ethyl, Propyl oder eine Polyethergruppe [-(O(R⁶-O)ₓ-]
   bedeuten.

### Variante D :

D. I Geminitenside der allgemeinen Formel (D.I) analog US 5,863,886 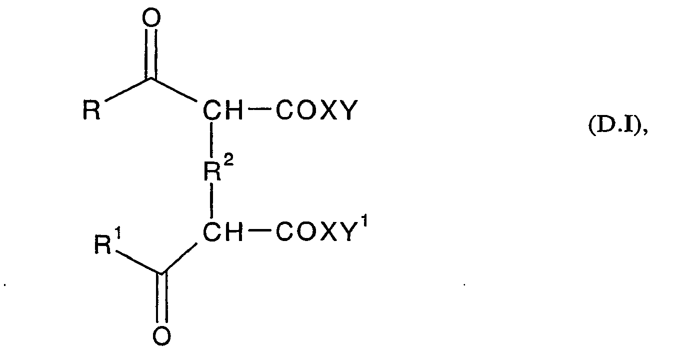 wobei die Substituenten unabhängig voneinander folgende Bedeutung haben:
   - R, R¹: C₅- bis C₃₀-Alkyl, verzweigt oder unverzweigt, gesättigt, gegebenenfalls bis zu 2-fach nicht-benachbart ungesättigt, hydroxysubstituiert oder perfluoriert;
   - R²: C₁-bis C₁₀- Alkylen, Arylen und hydroxysubstituierte Derivat, ein Polyether [-O(R⁴O)ₓ-], -S-, -SO₂- -O-, -S-S-, -O-R⁵-O- oder -S-R⁵-S-;
   - R⁴: C₂- bis C₄-Alkylen;
   - R⁵: C₁- bis C₁₀-Alkylen, Arylen oder Alkylarylen, -N(R⁶)- oder -(NR⁶)-R⁷-(NR⁶)- ;
   - R⁶: C₁- bis C₆-Alkyl;
   - R⁷: C₁- bis C₆-Alkyl, wobei R⁷ und R⁶ auch Teil eines heterocyclischen Ringes sein können;
   - X: Polyether [-O(R⁴O)ₓ-], wobei x eine Zahl von 1 bis 30 ist, -O-, NZ;
   - Z: C₁- bis C₁₀- Alkyl, Aryl, Alkylaryl oder H und
   - Y, Y¹: unabhängig voneinander H, -CH₂-COOH und Salze, ein Kohlenwasserstoffrest mit mindestens 2 Hydroxylgruppen, wie Erythrose, Threose, Ribose, Arabinose, Xylose, Fructose, Lyxose, Allose, Altrose, Glucose, Mannose, Galactose und ihre Mischungen.
D.II Geminitenside der allgemeinen Formel (D.II) 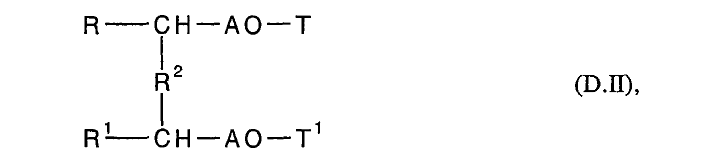 wobei die Substituenten unabhängig voneinander die für die allgemeine Formel (D.I) angegebene Bedeutung haben und
   - AO: -C(O)-, -C(O)- [-O(R⁴O)ₓ-], -CH₂- [-O(R⁴O)ₓ-], -CH₂-O-;
   - T, T¹: unabhängig voneinander -OM, -H, -CH₃, -C₂H₅, -SO₃M, -CH₂COOM, -C₂H₄-COOM, -C₃F₆-SO₃M, -O-P(O)(OM)₂ und
   - M: Alkyli, ½ Erdalkali, Ammonium, Mono-, Di-, Trialkanolammonium oder H bedeuten.
D.III Geminitenside der allgemeinen Formel (D.III) analog WO 96/16930 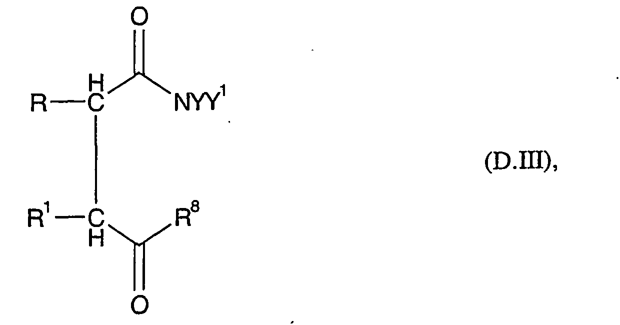 wobei die Substituenten unabhängig voneinander die für die allgemeinen Formeln (D.I) und (D.II) angegebene Bedeutung haben und
   - R⁸: NYY¹, -O(R⁴O)ₓH oder -O(R⁴O)ₓ-C(O)-CHR-CHR¹-C(O)NYY¹ bedeutet.
D.IV Geminitenside der allgemeinen Formel (D.IV) analog WO 96/25384 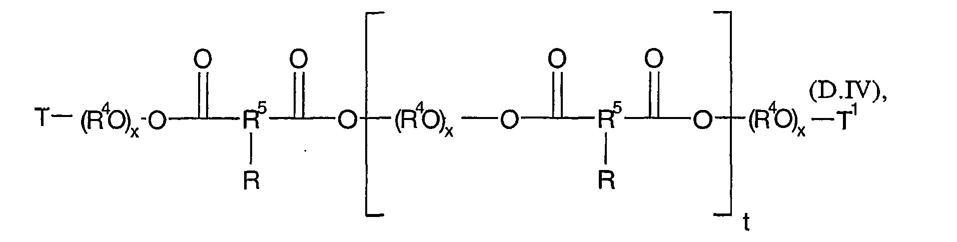 wobei die Substituenten die für die allgemeinen Formeln (D.I), (D.II) und (D.III) angegebene Bedeutung haben und
   - t: eine ganze Zahl von 1 bis 100, bevorzugt 1 bis 20, besonders bevorzugt 1 bis 4 bedeutet.

Die Terminologie "bis zu 2-fach nicht-benachbart ungesättigt" bezieht sich auf konjugierte Doppelbindungen.

### (a.2) Detergenskomponente mit schlecht schäumender Charakteristik,

Vorzugsweise werden als Detergenskomponente mit schlecht schäumender, vorzugsweise milder, Charakteristik folgende Verbindungen eingesetzt:
wasserlösliche Zuckertenside, acylierte Protein-Derivate, Sulfosuccinate, insbesondere Natrium-, -Mono- und -Di-alkanol-sulfobernsteinsäureester mit verzweigten oder unverzweigten, gesättigten oder 1 bis 3-fach nicht-benachbart ungesättigten Alkylresten im Bereich von C₆ bis C₁₈, oder Acyllactylate, insbesondere Natrium-, Kalium-, Magnesium- oder Calciumsalze der an der Hydroxylgruppe mit linearen oder verzweigten, gesättigten oder 1 bis 3-fach nicht-benachbart ungesättigten, cyclischen oder acyclischen C₆- bis C₂₄- Carbonsäuren veresterter monomerer Milchsäure oder deren Oligomeren, wobei der Oligomerisierungsgrad der Milchsäure vorzugsweise bei 1,1 bis 10, besonders bevorzugt bei 1,1 bis 4 liegt, oder Alkyl(poly)glucoside mit einem Oligomerisierungsgrad von 1,0 bis 10, vorzugsweise 1 bis 3, und Alkylresten, die verzweigt oder unverzweigt, gesättigt oder 1 bis 3-fach nicht-benachbart ungesättigt, cyclisch oder acylisch sind, und 6 bis 24 Kohlenstoffatome enthalten, oder Alkali-, Erdalkali-, Mono-, Di- und Trialkanolammonium-, Ammonium-, Mono-, Di-Trialkylammoniumsalze N-acylierter Aminosäuren, einschließlich ggf. auch teil-N-acylierter Oligo-/Poly-Aminosäuren, wie der Alkylisethionate, die Alkylreste mit 6 bis 24 Kohlenstoffen enthalten und verzweigt oder unverzweigt, gesättigt oder 1- bis 3-fach nicht-benachbart ungesättigt sind, oder Alkali-, Erdalkali-, Mono-, Di- und Trialkanolammonium-, Ammonium-, Mono-, Di-, Trialkylammoniumsalze der Acylsarcosinate, die Alkylreste mit 6 bis 24 Kohlenstoffen enthalten und verzweigt oder unverzweigt, gesättigt oder 1- bis 3-fach nicht-benachbart ungesättigt sind, oder Proteinkondensate mit C₆- bis C₂₄-Acylresten, die verzweigt oder unverzweigt, gesättigt oder 1- bis 3-fach nicht-benachbart ungesättigt sind, oder Betaine, die Alkylketten mit 6 bis 24 Kohlenstoffatomen enthalten, die verzweigt oder linear, gesättigt oder 1-bis 3-fach nicht-benachbart ungesättigt sein können. Bei den Betainen sind solche vom Amidoamin-Typ bevorzugt. Acylglutamate mit 6 bis 24 Kohlenstoffatomen in der Acylkette, die linear oder verzweigt, gesättigt oder 1- bis 3-fach nicht-benachbart ungesättigt sein können, eignen sich ebenfalls. Besonders bevorzugte Detergenskomponente für die erfindungsgemäßen Zusammensetzungen sind Acyllactylate, Alkylisethionate und/oder Acylglutamate bzw. deren Derivate.

### (b.2) Co-Amphiphile mit einem HLB- Wert von kleiner 6,

Zur Verwendung als Co-Amphile sind besonders bei Raumtemperatur (25°) feste Co-Amphiphile geeignet. Zu diesen bevorzugten Co-Amphiphilen gehören C₆- bis C₄₀-, bevorzugt C₈- bis C₂₄-Alkylalkohole, besonders bevorzugt Cetylalkohol oder Behenylalkohol, die verzweigt oder unverzweigt, gesättigt oder ein- bis dreifach nicht-benachbart ungesättigt, acyclisch oder alicyclisch sein können, nicht neutralisierte C₆- bis C₂₄-, bevorzugt C₈- bis C₂₂-Alkylcarbonsäuren, die verzweigt oder unverzweigt, gesättigt oder ein- bis dreifach nicht-benachbart ungesättigt, acyclisch oder alicyclisch sein können, Alkylarylderivate, Sorbitanester (C₆ bis C₂₂), Methylglucosidester (C₆ bis C₂₂), Zuckerester (C₆ bis C₂₂), Mono-, Di- und Triglyceride von C₆-bis C₂₂-Carbonsäuren oder Mischungen daraus, wobei Glycerin-mono-di-stearat besonders bevorzugt ist, verzweigt oder unverzweigt, gesättigt oder ein- bis dreifach nicht-benachbart ungesättigt, sowie Mono- und Diglyceride der vorgenannten Säuren, und ihre mit Milchsäure und/oder Zitronensäure weiter-veresterten Derivate, C₆-bis C₂₂-Polyglycerinester, C₆- bis C₂₂-Propylenglycolester, darüber hinaus Vitaminester (z.B. Vitamin E-acetat, Vitamin A-palmitat), Salicylsäure, Benzoesäure, Lecithine (aus Pflanzenölen oder tierischen Ursprungs). Besonders bevorzugt sind hier die Alkohole, Säuren und Mono-, Diglyceride der vorstehend genannten Carbonsäuren.

Bevorzugt sind hier Mischungen aus längerkettigen Alkoholen (ein C6- bis C40-Alkohol, aufsteigend bevorzugt C8- bis C24-, C14- bis C36- oder C14- bis C24- Alkohol), wie Cetylalkohol oder Behenylalkohol, Glycerinmono-di-stearat (GMS) und mit Zitronensäure verestertem Glycerinmonostearat oder als weitere Ausführungsform der Tensidzusammensetzung aus längerkettigen Alkoholen, wie Cetylalkhol oder Behenylalkohol oder Erucaalkohol, GMS und Stearinsäure, ganz besonders bevorzugt sind Mischungen aus Behenylalkohol, GMS und mit Zitronensäure verestertem Glycerinmonostearat.

Bevorzugt sind weiterhin eine oder mehrer der nachfolgenden Komponenten:
Polyethylenglycol-Derivate, mit breiter oder enger Homologenverteilung, Polyethylenpolypropylen- Blockcopolymere, Alkylpolyglucose (APG, mit einem DP von 1 bis 6), Alkylpolyglycerinderivate und Siliconcopolylole.

Die bevorzugten Geminitensid / Co-Amphiphil(e) Zusammensetzungen weisen unabhängig voneinander neben
- dem Geminitensid, vorzugsweise zu 5 bis 25 Gew.%, besonders bevorzugt 10 bis 20 Gew.% , bezogen auf die Zusammensetzung Geminitensid / Co-Amphiphil(e),
zumindest 2, vorzugsweise 3, der unten genannten Co-Amphiphil-Komponenten unterschiedlicher Charakteristik auf:
(a) einen oder mehrere längerkettige Alkohole:
   - ein C6- bis C40-, aufsteigend bevorzugt einen C8- bis C24-, C14- bis C36- oder C14- bis C24- Alkohol,
(b) eine oder mehrere längerkettige Säuren:
   - ein C6- bis C24-, bevorzugt C8- bis C22-, Carbonsäure,
(c) einen oder mehrere Ester/Teilester eines Polyols mit einer oder mehreren Mono- oder Poly-Carbonsäuren:
   - ein Sorbitan(C6- bis C22-)ester,
   - ein Methylglucosid(C6- bis C22-)ester,
   - ein Zucker(C6- bis C22-)ester,
   - ein Mono-, Di- und Triglycerid einer C6- bis C22- Carbonsäure,
   - ein mit Milchsäure oder Zitronensäure verestertes Derivat der Mono- und Diglyceride einer C6- bis C22-Carbonsäure,
   - ein Polyglycerin(C6- bis C22-)ester,
   - ein Propylenglycol(C6- bis C22-)ester,
   - ein Vitaminester,
(d) sowie folgende weitere Co-Amphiphil-Komponenten:
   - Salicylsäure,
   - Benzoesäure und / oder
   - Lecithin.

Nach einer weiteren Ausführungsform sind bevorzugt zumindest 2, vorzugsweise zumindest 3, der unten genanten Komponenten Co-Amphihil:
- 30 bis 50 Gew.% einer oder mehrerer längerkettiger ( >= C8) Alkohole (a),
- 30 bis 50 Gew.% eines Glycerin-Derivates wie eines Mono-, Di- und Triglycerides einer C6- bis C22- Carbonsäure, oder eine Verbindung ähnlichen HLB's,
- 5 bis 25 Gew.%, vorzugsweise 10 bis 20 Gew.%, einer mit Milchsäure oder einem Zitronensäure verestertem Derivat der Mono- und Diglyceride einer C6- bis C22-Carbonsäure und/oder eine C6- bis C22-Carbonsäure,
jeweils bezogen auf die Zusammensetzung Geminitensid / Co-Amphiphil.

Vorzugsweise ist der längerkettige Alkohol zumindest eines der eingesetzten Co-Amphiphile und ein Ester eines Polyols mit einer oder mehreren Mono- oder Poly-Carbonsäuren, vorzugsweise mit 6 bis 22 Kohlenstoffatomen, die (eine) weitere eingesetzte Co-Amphiphil-Komponente.

Kommen z.B. 5 Co-Amphiphile zum Einsatz enthält die erfindungsgemäße Zusammensetzung neben dem Geminitensid zu 5 bis 25, bevorzugt 5 bis 20 Gew.%, vorzugsweise

| | |
|---|---|
| Co-Amphiphil 1 | langkettiger Alkohol (a) zu 20 bis 50 %, bevorzugt 20 bis 35 Gew.%, |
| Co-Amphiphil 2 | einen Ester/Teilester eines Polyols mit einer oder mehreren Mono-oder Poly-Carbonsäuren (c), insbesondere GMS oder Verbindung mit vergleichbaren HLB, zu 20 bis 50, bevorzugt 20 bis 35 Gew.%, |
| Co-Amphiphil 3 | zu 5 bis 25 %, bevorzugt 10 bis 20 Gew.%, |
| Co-Amphiphil 4 | zu 5 bis 25 %, bevorzugt 10 bis 20 Gew.% und |
| Co-Amphiphil 5 | zu 5 bis 25 %, bevorzugt 10 bis 20 Gew.%. |

Ein besonders bevorzuge Ausführungsform der Erfindung weist folgende Zusammensetzung (a.1 + a.2) auf:

| | | |
|---|---|---|
| Geminitensid | 5 bis 15 | Gew.%, |
| Glycerinmono-distearat | 30 bis 40 | Gew.%, |
| Behenylalkohol | 35 bis 45 | Gew.% und |
| Glycerylstearatcitrat | 10 bis 20 | Gew.%. |

### (b.3) Hydrophobe Komponente,

Unter hydrophober Komponente versteht man Mono-, Di- und Triglyceride, Esteröle, Paraffine, Cycloalkane, cyclische und acyclische Siliconöle und funktionalisierte Siliconöle, langkettige Alkohole mit Kettenlängen > C14, Isoprenderiviate, Alkydharze, C₆- bis C₄₀-, bevorzugt C₈- bis C₂₄-Alkylakohole, besonders bevorzugt Cetylalkohol oder Behenylalkohol, die verzweigt oder unverzweigt, gesättigt oder ein- bis dreifach nicht-benachbart ungesättigt, acyclisch oder alicyclisch sein können, nicht neutralisierte C₆- bis C₂₄-, bevorzugt C₈- bis C₂₂-Alkylcarbonsäuren, die verzweigt oder unverzweigt, gesättigt oder ein- bis dreifach nicht-benachbart ungesättigt, acyclisch oder alicyclisch sein können, Alkylarylderivate, Sorbitanester (C₆ bis C₂₂), Methylglucosidester (C₆ bis C₂₂), Zuckerester (C₆ bis C₂₂), Mono-, Di- und Triglyceride von C₆- bis C₂₂-Carbonsäuren oder Mischungen daraus, wobei Glycerin-mono-di-stearat besonders bevorzugt ist, verzweigt oder unverzweigt, gesättigt oder ein- bis dreifach nicht-benachbart ungesättigt, sowie Mono- und Diglyceride der vorgenannten Säuren, und ihre mit Milchsäure und/oder Zitronensäure weiter-veresterten Derivate, C₆- bis C₂₂-Polyglycerinester, C₆- bis C₂₂-Propylenglycolester, darüber hinaus Vitaminester ( z.B. Vitamin E-acetat, Vitamin A-palmitat), Salicylsäure, Benzoesäure, Lecithine (aus Pflanzenölen oder tierischen Ursprungs). Besonders bevorzugt sind hier die Alkohole, Säuren und Mono-, Diglyceride der vorstehend genannten Carbonsäuren, Ester von verzweigten und unverzweigten, gesättigten und ein- bis dreifach ungesättigten Alkoholen mit Milch-, Äpfel-, Salicyl-, Benzoe- und Weinsäure.

### (b.4) Nichtionische Teniside

Unter nichtionischen Tensiden im Sinne der Erfindung sind die Alkoxylate von verzweigten oder linearen, gesättigten oder ein- bis dreifach ungesättigten C6- bis C22-Alkoholen oder Alkylpolyglycoside mit Polymerisationsgraden ≥1 oder Sorbitanester oder Sorbitanesterethoxylate oder C6- bis C22- linear oder verzweigte Alkylpolyglyceride mit wenigstens 2 Glyceryleinheiten oder Alkoxylate der Mono- und Difettsäureglyceride oder deren Gemische oder N,N'-Diacylalkylendiaminalkoxylate oder Ethylenoxid-Propylenoxid Block-Co-Polymere oder N-Alkylpyrrolidonderivate oder Polyvinylalkoholderivate zu verstehen. Unter Alkoxyden sind Ethylenglykolether, Propylenglykolether und deren Kombinationen blockweise oder willkürlich kombiniert zu verstehen.

### (c.2.1) Alkohole

Bevorzugte Alkohole sind C2- bis C4-Alkohole, etwa Ethanol und Propanol.

### (c.2.2) Polyglykole

Bevorzugte Polyglykole sind Oligomere von Polyolverbindungen mit vorzugsweise 2 bis 4 Kohlenstoffatomen und vorzugsweise 2 oder mehr Hydroxygruppen, bezogen auf das Monomer als Kettenbaustein. Die Oligomere können als Start- bzw. Endgruppe 1 oder 2 freie Hydroxygruppen aufweisen oder aber auch vollständig veretherte Start- bzw. Endgruppen aufweisen. Als Startgruppe können C1- bis C8- Mono- oder Polyole eingesetzt werden. Als Endgruppe C1- bis C8-Monohydroxy-Alkohole. Vorzugsweise weisen die Polyglykole ein Molekulargewicht bis < 25 000 g/mol auf. Beispielhaft genannt seien Polyethylen-, Polypropylen-, und Polybutylenglycole.

### (c.2.3) Polyole

Die Polyolen weisen vorzugsweise 2 bis 10 Kohlenstoffatome im (ggf. verzweigten) Alkylenteil und vorzugsweise 2 bis 50, besonders bevorzugt 2 bis 6, Hydroxylgruppen auf. Geeignete Polyole sind z.B. Alkylenglykole wie Ethylen-, Propylen-, Butylen-, Pentylen- sowie Hexylenglykol sowie ihre jeweiligen Isomeren (z.B. Neopentylglykol), wie auch Triole, wie Glycerin, und höhere, wie Trimethylolpropan, Pentaerythrit, Polyglycerine, Glucoside und Polyglucoside, Saccharide und ihre jeweiligen Alkylderivate und Polyvinylalkohole und Mischungen. Geeignet sind auch Polyvinylalkohole.

### (c.4) Viskositätsregulator

Als Viskositätsregulator sind in Wasser quellende hydrophile Polymere geeignet. Geeignet sind etwa nicht-vernetzte Polymere. Beispielhaft seien genannt: Xanthan Gum, lineare Acrylsäure-Polymere, Pectine, Saccharide, Cellulosederivate, Caragenan und Gummi Arabicum.

### (c.5) Vernetzte Polymere

Als vernetzte Polymere lassen sich quervernetzte Polyacrylsäurederivate und Copolyacrylsäurederivate, wie z. B. Copoylmerisate mit Maleinsäure verwenden.

### (d.1) Feststoffpartikel

Unter Feststoffpartikeln im Sinne der Erfindung sind Partikel zu verstehen, die in den erfindungsgemäßen O/W-Emulsion im wesentlichen unlöslich sind. Die Feststoffpartikel weisen vorzugsweise im wesentlichen mittlere Partikelgrößen bezogen auf die Primärpartikel bis 20 µm, vorzugsweise von 0,01 bis 5 µm auf.

Eine besonders feine Verteilung des Festkörperpartikel und seine Stabilisierung gegenüber Agglomeration wird erzielt werden, wenn die Festkörperpartikel vordispergiert der Zusammensetzung zugesetzt sind, z.B. in einem Polyol gemäß Komponente (c.2.3). Hierbei können die Feststoffpartikel in Form einer Paste eingesetzt sein.

In der erfindungsgemäßen O/W-Emulsion können etwa bis 8 Gew.% Titandioxid-Partikel so gut dispergiert werden, dass es beim Verteilen der O/W-Emulsion auf der Haut nicht zum Weißeln kommt. Gleichzeitig ist die Emulsion stabil. So können die erfindungsgemäßen O/W-Emulsionen einem 5-fachen Gefrier- Tau-Zyklus (-18 °C - > 40 °C) und einer zwei monatigen Lagerung bei Raumtemperatur oder bei 50 °C ausgesetzt sein, ohne dass Instabilitäten beobachtet werden können.

### Geeignete Feststoffpartikel sind:

Titandioxid, amphiphil oder hydrophob beschichtet, Zinkoxid, Effektpigmente, die ebenfalls beschichtet sein können, Silica, Bornitride, Ruße, Eisen- und Aluminiumoxide, die jeweils amphiphil oder hydrophob gecoatet sein können und besonders bevorzugt solche die mit Siliconderivaten beschichtet sind, Alumosilicate, Tonerden, wie Bentonite, Mica, anorganische Perlglanzpigmente und Metallpulver und Metalloxide, wie Zinnoxid, Carmine, Eisenferrocyanide, Bariumsulfat, Wismuthoxichlorid, wie auch organische Pigmente wie Farbstoffpigmente und Lichtschutzpigmente wie 2,2'-Methylene-bis- {6-(2H-benzotriazole-2-yl)-4-(1,1,3,3,-tetramethylbutyl)-phenol} zu verstehen.

Besonders vorteilhaft hat sich die Kombination Titandioxid mit amphiphilem oder hydrophobem Coating und Salicylsäureester erwiesen. Die Alkoholgruppe weist vorzugsweise 6 bis 22 Kohlenstoffatome auf. Der erzielbare Lichtschutzfaktor ist deutlich höher als bei der Kombination der beiden Komponenten zu erwarten wäre. COSMACOL® ESI ist ein geeigneter handelsüblicher Salicylsäureester.

### Verwendung

Die erfindungsgemäßen O/W-Emulsionssprays können in der Kosmetik, beispielsweise alternativ zu Hautpflegecremes oder Hautpflegelotionen oder als im Sonnenschutz als Alternative zu den gebräuchlichen Lotionen eingesetzt werden. Sie weisen dann Lichtschutzfaktoren von 2 bis 60 auf. Ebenso ist eine Verwendung in der dekorativen Kosmetik beispielsweise bei Verwendung von Effektpigmenten oder als flüssige / sprühfähige Foundation möglich.

Darüber hinaus können die erfindungsgemäßen sprühfähigen O/W-Emulsionen auch als Lederpflegemittel, Möbelpflegemittel, Polierhilfsmittel bei der Kfz-Pflege, Polierhilfsmittel bei der Herstellung von Siliciumwafem, als Farben für Oberflächenlackierungen, sowohl für saugende als auch für nichtsaugende Oberflächen eingesetzt werden.

Die erfindungsgemäßen Zusammensetzungen können als multifunktionelle Zubereitung in der Kosmetik zur Haut- und Haarpflege, bzw. -reinigung, als Haushaltsreiniger, als Möbelpflege oder -reinigungsmittel, als Textilpflege oder -reinigungsmittel, als Teppichpflege- oder -reinigungsmittel, im Pflanzenschutz als Herbizid, Insektizid oder Arachnizid und zur industriellen und institutionellen Reinigung eingesetzt werden.

### Anwendung Schäume

Zur Erzeugung einer schaumartigen Emulsion mit einer geeigneten durch Fingerdruck betriebenen Pumpe (nicht unter Anwendung eines unter Druck stehenden Treibmittels), wie sie z.B. Airspray International anbietet, muss die Emulsion in einem geeigneten Viskositätsbereich liegen. Da es sich hierbei um sehr niedrigviskose Emulsionen handelt, treten hier zunächst dieselben Schwierigkeiten hinsichtlich der Herstellung, der Stabilität wie auch der Flexibilität dieser O/W-Emulsionen wie auch bei sprühbaren O/W-Emulsionen auf. Die Möglichkeit einer zusätzlichen Stabilisierung durch Erhöhung der Viskosität entfällt. Da einfache sprühbare O/W-Emulsionen nicht gut anschäumbar sind und wenn überhaupt, dann nur einen wenig stabilen und nicht besonders cremigen Schaum bilden, bestand die Aufgabe eine stabile niedrigviskose O/W-Emulsion zu formulieren, die mit Hilfe zusätzlichen Tensides gut anschäumbar sind, ohne gleichzeitig an Stabilität und Flexibilität hinsichtlich der Bandbreite and möglichen Ölpolaritäten zu verlieren.

Überraschend ist es möglich diese Aufgabe zu lösen, indem man nach Herstellung eines Sprays einfach eine 5 bis 30 %ige wässrige Lösung des stark schäumenden Tensids oder eine Zubereitung enthaltend dieses der Formulierung hergestellt aus den Phasen (a) und (b) hinzugibt. Das erfindungsgemäß zugesetzte Tensid sollte zwischen 0.1 und 3.0 Gew.%, bevorzugt 0.5 bis 2.0 % Gew.% und besonders bevorzugt 1.0 bis 2.0 Gew.% jeweils, bezogen auf die O/W-Emulsion enthalten sein.

Als schäumendes Tensid sind besonders geeignet Alkali-, Erdalkali- und Ammonium- und Alkanol und Alkylammoniumsalze der Acylglutamate (C6 bis C22, gesättigt oder ein- bis dreifach ungesättigt, verzweigt oder linear), Alkali-, Erdalkali- und Ammonium- und Alkanol und Alkylammoniumsalze der Alkyl- oder Alkenylisethionate, Alkyl- oder Alkenyllactate, Mischungen aus Alkyllactylatsalzen und einem oder mehreren Geminitensiden (siehe oben), z.B. CERALUTION® F, Alkylamidopropylbetaine, N-Acylsarcosinate, und anderer schäumender N-acylierter Aminosäuren bzw. deren Salzen, Salze von Alkyl- oder Dialkylsulfosuccinaten. Auch das schäumende Tensid kann Geminitenside enthalten, wobei sich die hier zugesetzten Mengen zu den in Phase (a) und (b) enthaltenen Mengen addieren.

Herstellbar sind diese Schäume, indem zuerst die Basis O/W-Emulsion, die auf einem nichtionischen PIT-System oder auf einem anionischen Tensid, wie Glyceryl Stearat Citrat oder einem Phosphorsäureesterderivat oder auf Geminitensid-Mischungen beruhen können, entsprechend dem System hergestellt werden und anschließend bei 40 bis 20 °C, bevorzugt 35 °C, das schäumende Tensid, vorzugsweise als Lösung zugesetzt wird.

Die erfindungsgemäßen Verhältnisse liegen hier bei 70 bis 95 Gew. %, bevorzugt 80 bis 95 Gew. %, der niedrigviskosen Emulsion und 5 bis 30 Gew. %, bevorzugt 10 bis 20 Gew. % einer 1 bis 15 Gew. %igen, bevorzugt einer 1 bis 3 %igen Tensidlösung.

### Experimentelles

Mild im Sinne der Erfindung bedeutet, daß die entsprechenden Verbindungen / Zusammensetzung kennzeichnungsfrei (z.B. gemäß Richtlinie 67-548-EWG, Gefahrstoffverordnung) hinsichtlich ihres Irritationspotentials für Haut und Augen sind.

Unter der Eigenschaft "mit schlecht-schäumender Charakteristik" ist im Sinne der Erfindung zu verstehen, daß diese Tenside in ihrer Eigenschaft als weitere Detergenskomponente zwei von drei der unten aufgeführten Werte bei der Beurteilung ihres Schaumbildungsverhaltens unterschreiten.

Die Kriterien sind:
- die Schaumlamellendicke in mm direkt nach der Schaumherstellung,
- der Blasenzahl pro Bildfläche bei 100facher Vergrößerung direkt nach der Herstellung, beide durch die Mikroskopie des Schaumes bestimmt, und
- das Anschäumverhalten im Handversuch.

Versuchsdurchführung zur Bestimmung der Eigenschaft "mit schlecht-schäumender Charakteristik":

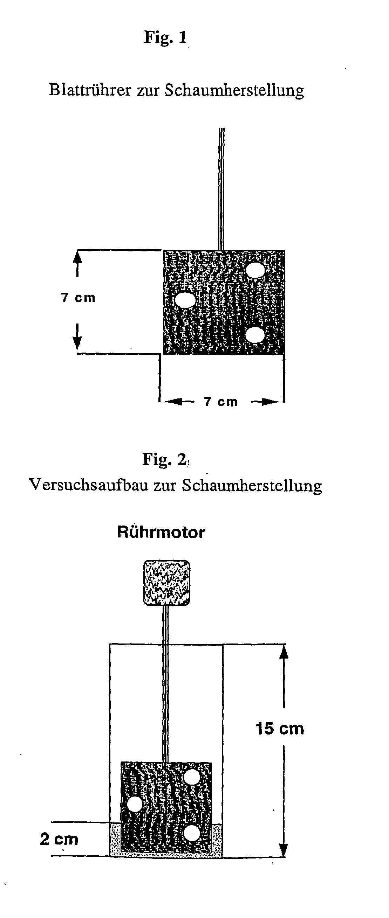

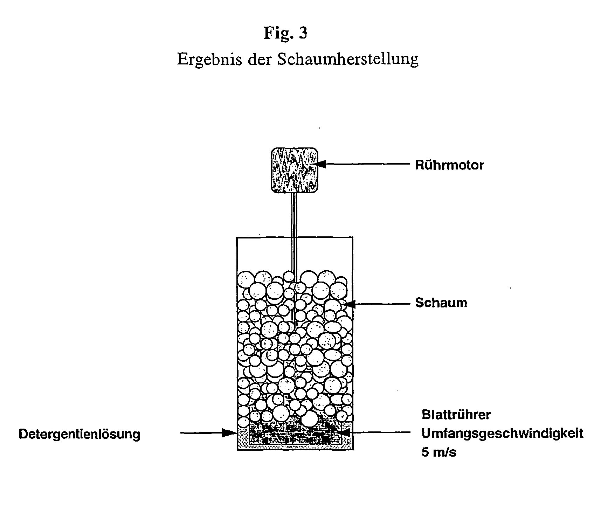

8 Gew.-% des zu charakterisierenden Tensids werden in demineralisiertem Wasser gelöst. Die zu untersuchende Tensidlösung wird 10 min. bei 1500 U/min mit Apparatur mit Blattrührer gemäß Figur 1/3 bis 3/3 gerührt, wobei sich die Lösung etwas erwärmt (von Raumtemperatur auf ca. 35°C). Nach 10 Minuten Rührzeit wird der entstandene Schaum von oben abgeschöpft und direkt mikroskopiert - hierbei wird die Lamellendicke in mm gemessen und die Anzahl der Schaumblasen im Bildausschnitt bestimmt.

Neben der durch Rühren erzeugten Schaumqualität wird auch das Anschäumverhalten eines Tensids unter fließendem kalten Leitungswasser beurteilt. Hierzu werden 2 g Tensid auf die Innenhandflächen verteilt und unter fließendem Wasser gleichmäßig verrieben. Die Schaumqualität wird in vier Stufen beurteilt, 0 = keine Schaumbildung, 1 = mäßige Schaumbildung, 2 = gute Schaumbildung und 3 = sehr gute Schaumbildung.

Als schlecht schäumend werden Tenside beurteilt, wenn sie die Grenzen von mindestens zwei der drei unten genannten Kriterien unterschreiten, d.h.
- Lamellendicken kleiner oder gleich 16 mm aufweisen oder
- kleiner oder gleich 16 Blasen im Bildausschnitt aufweisen oder
- im Anschäumverhalten mit 1 oder schlechter beurteilt werden.

Als gut schäumend werden Tenside beurteilt, wenn sie alle drei Kriterien mit Werten von ≥ 20 mm in der Lamellendicke, ≥ 20 Blasen im Bildausschnitt (beides direkt nach dem Anschäumen) und ein mit 3 beurteiltes Anschäumverhalten aufweisen.

Das verwendete Verfahren zur Schaumherstellung ist in Fig. 1 bis 3 schematisch dargestellt. Die Zeichnung zeigt schematisch in Fig. 1 den verwendeten Blattrührer zur Schaumherstellung, in Fig. 2 den Versuchsaufbau zur Schaumherstellung - jeweils mit den Größenangaben in cm, wobei H die Höhe der ungeschäumten Lösung ist -, und in Fig. 3 den Zustand nach Aufschäumen bei einer Umfangsgeschwindigkeit des Blattrührers von 5 m/sec. (S = Schaum, D = Detergenslösung). Nach 10 Minuten Rührzeit wurde der entstandene Schaum von oben abgeschöpft und sowohl direkt als auch nach 2, 5 und 15 Minuten mikroskopiert.

Mild im Sinne der Erfindung bedeutet, daß die entsprechenden Verbindungen / Zusammensetzung kennzeichnungsfrei (z.B. gemäß Richtlinie 67-548-EWG, Gefahrstoffverordnung) hinsichtlich ihres Irritationspotentials für Haut und Augen sind.

### Herstellung

Die Herstellung der erfindungsgemäßen sprühbaren Emulsionen erfolgt in zumindest zwei Schritten, wie nachfolgend beispielhaft beschrieben: Bei 60 °C wird zur einem Teil der hydrophilen Phase (a), zu der ggf. der Viskositätsregulator als vorteilhaft in Wasser vorgequollene lineares Polymer gegeben worden ist, die Phase (b) gegeben und solange homogenisiert bis >95 %, vorzugsweise >98 % der Öltröpfchen kleiner 1 mm, vorzugsweise kleine 10 µm im Durchmesser groß sind.

Durch Korrektur des Wasseranteils (Erhöhung, Senkung) wird die Viskosität so eingestellt, dass möglichst kurz homogenisiert werden muss. Anschließend wird auf 35 bis 40 °C abgekühlt und der Rest der Wasserphase, gegebenenfalls Konservierungsmittel und Parfüm, sowie andere leicht flüchtige Formulierungsanteile, wie z.B. Cyclomethicone, und das in Polyol, bevorzugt Butylenglykol, vordispergierte Pigment eingerührt. Die Auswahl der geeigneten Komponente (a.1,a.2;b.1,b.2) wird gewährleistet, dass die Partikel nicht agglomerieren. Die Sprühcharakteristik wird überraschend nicht vom Feststoffpartikel-Gehalt beeinflusst.

Die Herstellung der erfindungsgemäßen schäumenden Emulsionen erfolgt in zumindest drei Schritten, wie nachfolgend beschrieben, wobei zunächst eine sprühfähige Emulsion, wie oben beschrieben hergestellt wird: Anschließend wird eine verdünnte wässrige Lösung eines schäumenden, vorzugsweise stark schäumenden Tensides zugemischt. Das Produkt wird abgekühlt.

Die Herstellung der erfindungsgemäßen sprühfähigen Antitranspirant - Emulsionen erfolgt in zumindest drei Schritten, wie nachfolgend beschrieben, wobei zunächst eine sprühfähige Emulsion, wie oben beschrieben hergestellt wird: Anschließend wird eine wässrige Lösung oder Suspension oder eine Alkylenglykol-Lösung oder Suspension (vorzugsweise Propylen- oder Butylenglykol des Metall-Chloro-Hydrates eingerührt.

| **Beispiel** | | **A** | **B** | **C** | **D** | **E** | **F** | **G** | **H** |
|---|---|---|---|---|---|---|---|---|---|
| **Handelsname** | **CTFA/INCI** | **[Gew.-%]** | | | | | | | |
| | **Phase B bzw. Zugabe zu** | | | | | | | | |
| Ceralution H | Behenyl Alcohol, Glyceryl | 1,00 | 1,00 | 1,00 | 1,00 | 1,00 | 1,00 | 1,00 | 1,00 |
| | Stearate, Glyceryl Stearate Cit- | | | | | | | | |
| | rate, Sodium Dicocoylethyl- | | | | | | | | |
| | enediamine PEG-15 Sulfate | | | | | | | | |
| Marlipal O13/120 | Isotrideceth-12 | 2,00 | 0,00 | 0,00 | 2,00 | 0,00 | 0,00 | 0,00 | 2,00 |
| Marlipal 1618/25 | Ceteth-25 | 0,00 | 2,00 | 2,00 | 0,00 | 2,00 | 2,00 | 2,00 | 0,00 |
| Miglyol 812 N | Caprylic/capric triglyceride | 25,00 | 25,00 | 25,00 | 25,00 | 25,00 | 5,00 | 14,00 | 25,00 |
| Cosmacol ESI | | | 3,00 | | | | | | |
| | C12-15 Alkyl benzoate | 2,90 | 0,00 | 0,00 | 0,00 | 0,00 | 2,50 | 0,00 | 0,00 |
| | Cyclomethicone | 0,00 | 0,00 | 0,00 | 0,00 | 0,00 | 4,00 | 0,00 | 0,00 |
| | Triethyl citrate | 0,00 | 0,00 | 0,00 | 0,00 | 0,00 | 4,00 | 0,00 | 0,00 |
| | Octocrylene | 0,00 | 0,00 | 0,00 | 0,00 | 0,00 | 5,00 | 4,00 | 0,00 |
| | Octyl methoxycinnamate | 0,00 | 0,00 | 0,00 | 0,00 | 0,00 | 5,00 | 0,00 | 0,00 |
| | Butyl methoxydibenzoylmethane | 0,00 | 0,00 | 0,00 | 0,00 | 0,00 | 1,00 | 0,00 | 0,00 |
| | Tocopherol acetate | 0,00 | 0,00 | 0,00 | 0,00 | 0,00 | 0,50 | 0,00 | 0,00 |
| Uvinul T 150 | Octyl triazone | 0,00 | 0,00 | 0,00 | 0,00 | 0,00 | 0,00 | 4,00 | 0,00 |
| | **Phase A bzw. Zugabe zu** | | | | | | | | |
| demin. Wasser | | 10,00 | 10,00 | 10,00 | 10,00 | 10,00 | 10,00 | 10,00 | 10,00 |
| Ceralution F | Sodium Lauroyl Lactylate, So- | 1,50 | 2,00 | 2,50 | 1,50 | 2,00 | 1,50 | 1,50 | 2,00 |
| | dium Dicocoylethylenediamine | | | | | | | | |
| | PEG-15 Sulfate | | | | | | | | |
| Pricerine 9091 | Glycerin | 6,00 | 6,00 | 6,00 | 6,00 | 6,00 | 6,00 | 6,00 | 6,00 |
| Keltrol | Xanthan gum | 0,10 | 0,10 | 0,10 | 0,10 | 0,10 | 0,00 | 0,05 | 0,10 |
| | Hydroxyethylcellulose | 0,00 | 0,00 | 0,00 | 0,00 | 0,00 | 0,20 | 0,00 | 0,00 |

| | **Phase C** | | | | | | | | |
|---|---|---|---|---|---|---|---|---|---|
| demin. Wasser | Aqua | 34,50 | 29,90 | 27,40 | 33,40 | 27,90 | 25,80 | 28,20 | 32,90 |
| Ethanol | Alcohol | 7,00 | 6,00 | 6,00 | 6,00 | 6,00 | 6,00 | 6,00 | 6,00 |
| | Phenylbenzimidazole-5-sulfonic | 0,00 | 0,00 | 0,00 | 0,00 | 0,00 | 4,50 | 4,25 | 0,00 |
| | acid | | | | | | | | |
| | Sodium hydroxide (10 %) | 0,00 | 0,00 | 0,00 | 0,00 | 0,00 | 6,00 | 6,00 | 0,00 |

| | **Phase D** | | | | | | | | |
|---|---|---|---|---|---|---|---|---|---|
| ZnO/40% in BG | | 0,00 | 0,00 | 0,00 | 10,00 | 20,00 | 0,00 | 0,00 | 0,00 |
| UV-Sperse T40/BG | | 10,00 | 15,00 | 20,00 | 0,00 | 0,00 | 10,00 | 10,00 | 0,00 |
| | Silica | 0,00 | 0,00 | 0,00 | 0,00 | 0,00 | 0,00 | 0,00 | 10,00 |
| | Butylene glycol | 0,00 | 0,00 | 0,00 | 0,00 | 0,00 | 0,00 | 0,00 | 2,00 |
| | Pentylene glycol | 0,00 | 0,00 | 0,00 | 5,00 | 0,00 | 0,00 | 3,00 | 3,00 |
| **Summe**: | | 100,00 | 100,00 | 100,00 | 100,00 | 100,00 | 100,00 | 100,00 | 100,00 |
| **PSA** | | | | | | | | | |
| median o. sonic | | 0,32 | 0,34 | 0,37 | 0,34 | 0,35 | 0,34 | 0,36 | 0,33 |
| [*µ*m] | | | | | | | | | |
| < 1*µ*m [%] | | 98,6 | 99,2 | 98,7 | 99,6 | 98,3 | 98,9 | 99,4 | 98,9 |
| median m. sonic | | 0,33 | 0,34 | 0,37 | 0,34 | 0,35 | 0,34 | 0,36 | 0,33 |
| [*µ*m] | | | | | | | | | |
| < 1*µ*m [%] | | 98,7 | 99,2 | 98,5 | 99,7 | 98,2 | 99,1 | 99,4 | 98,8 |

| | | | | | | | | | |
|---|---|---|---|---|---|---|---|---|---|
| Herstellung: Phasen A und B auf 70°C erwärmen, Phase A zu B geben und homogenisieren bis PSA < 0.40µm; auf 35°C abkühlen und nacheinander Phase C und D einhomogenisieren. BG = Butylene glycol; Gemini: INCI: Sodium Dicocoylethylenediamine PEG-15 Sulfate (Struktur (A.1) mit R¹ = R³= C₁₁H₂₃-/C₁₃H₂₇, R²=C₂H₄, X = Y = (C₂H₄O-)x(C₃H₆O-)y-SO₃Na mit x = 15, y = 0); Ceralution H: (Behenyl Alcohol: 30 - 50 %, Glyceryl Stearate: 30 - 50 %, Glyceryl Stearate Citrate: 10 - 20 %, Gemini: 10 - 20 %); Ceralution F : (Natrium Lauroyl Lactylate: 30 - 50 %, Gemini: 30 - 50 %,Wasser: 15 %) | | | | | | | | | |

| Beispiel: | | **Schäumende Emulsion J** | **Schäumende Emulsion K** | **Sprühemulsion mit Al-Chloro-Hydrat L** |
|---|---|---|---|---|
| **Handelsname** | **CTFA/INCI** | **[Gew.-%]** | | |
| | **Phase B** | | | |
| Ceralution | Behenyl Alcohol, | 0,75 | 0,78 | 0,5 |
| H | Glyceryl Stearate, | | | |
| | Glyceryl Stearate | | | |
| | Citrate, Sodium Di- | | | |
| | cocoylethylenedia- | | | |
| | mine PEG-15 Sulfate | | | |
| | Steareth-100 | 0,00 | 0,00 | 1,00 |
| Marlipal | Isotrideceth-12 | 1,49 | 0,78 | 0,00 |
| O13/120 | | | | |
| Miglyol 812 | Caprylic/capric | 37,31 | 5,16 | 25,00 |
| N | triglyceride | | | |
| | Hydrogenated Co- | 0,00 | 2,35 | 1,05 |
| | coglycerides | | | |
| Cosmacol | C12-15 Alkyl ben- | 0,00 | 5,63 | 2,8 |
| EBI | zoate | | | |
| | Cyclomethicone | 0,00 | 3,91 | 2,5 |
| | Triethyl citrate | 0,00 | 0,00 | 0,00 |
| | Ethylhexylsalicylate | 0,00 | 0,83 | 0,00 |
| | Octyl methoxycin- | 0,00 | 10,95 | 0,00 |
| | namate | | | |
| | Benzophenone-3 | 0,00 | 1,56 | 0,00 |
| | Butyl methoxy- | 0,00 | 0,78 | 0,00 |
| | dibenzoylmethane | | | |

| | **Phase A** | | | |
|---|---|---|---|---|
| demin. H₂O | | 4,26 | 9,32 | 10,00 |
| Ceralution F | Sodium Lauroyl | 0,75 | 1,56 | 0,50 |
| | Lactylate, Sodium | | | |
| | Dicocoylethyl- | | | |
| | enediamine PEG-15 | | | |
| | Sulfate | | | |
| Pricerine | Glycerin | 8,96 | 7,82 | 6,00 |
| 9091 | | | | |
| Keltrol | Xanthan gum | 0,37 | 0,31 | 0,15 |

| | **Phase C** | | | |
|---|---|---|---|---|
| demin. H₂O | Aqua | 32,25 | 35,28 | 34,5 |
| Ethanol | Alcohol | 11,94 | 10,95 | 8,00 |

| | **Phase D** | | | |
|---|---|---|---|---|
| | Sodium lauroyl | 1,92 | 2,01 | 0,00 |
| | glutamate * | | | |
| | Sodium Lauroyl- | 0,00 | 0,00 | 0,00 |
| | sarcosinat* | | | |
| | Aluminium | 0,00 | 0,00 | 8,00 |
| | Chlorohydrex PG | | | |
| **Summe**: | | 100,00 | 100,00 | 100,00 |
| zusätzliches Wasser bei Zugabe | | 58 | 63 | |
| schäumendem Tensid * | | | | |
| Gehalt an schäumendem Tensid | | 1,2 | 1,2 | 0,00 |
| (100 % Aktivgehalt) | | | | |

| **PSA** | | | | |
|---|---|---|---|---|
| median o. sonic [µm] | | 0,36 | 0,36 | 0,35 |
| | < 1µm [%] | 99,4 | 99,2 | 99,5 |
| median m. sonic [µm] | | 0,36 | 0,34 | 0,34 |
| | < 1µm [%] | 99,4 | 99,2 | 99,6 |

| | | | | |
|---|---|---|---|---|
| Herstellung: - Phasen A und B auf 70°C erwärmen, Phase A zu B geben und homogenisieren bis PSA < 0.40 µm; auf 35°C abkühlen und nacheinander Phase C und D einhomogenisieren BG = Butylene glycol; PG = Propylene Glycol, Gemini: INCI: Sodium Dicocoylethylenediamine PEG-15 Sulfate (Struktur (A.1) mit R¹ = R³= C₁₁H₂₃-/C₁₃H₂₇, R²=C₂H₄, X = Y = (C₂H₄O-)x(C₃H₆O-)y-SO₃Na mit x = 15, y = 0) Ceralution H: (Behenyl Alcohol: 30 - 50 %, Glyceryl Stearate: 30 - 50 %, Glyceryl Stearate Citrate: 10 - 20 %, Gemini: 10 - 20 %); Ceralution F : (Natrium Lauroyl Lactylate: 30 - 50 %, Gemini: 30 - 50 %,Wasser: 15 %) Beispiel J: Die schäumende Emulsion lässt sich ebenso durch einfaches Vermischen einer bereits erfindungsgemäß hergestellten Sprühemulsion mit einer verdünnten Tensidlösung, im Beispiel 70 % Sprühemulsion, 30 % Tensidlösung 10%ig, herstellen. | | | | |

## Patentansprüche

1. Öl in Wasser Emulsion (O/W-Emulsion) mit einer Viskosität von kleiner 2000 mPas, gemessen bei 298 K, herstellbar unter Zusammenbringen
(a) einer hydrophilen Phase enthaltend:
(a.1) eines oder mehrere Geminitensid-Verbindungen und
(a.2) eine Detergenskomponente mit schlecht schäumender Charakteristik, im Verhältnis Geminitensid-Verbindung (a.1) zu Detergenskomponente (a.2) von 1 zu 100 bis 10 zu 1 und
(a.3) 1 bis 15 Gew.% Wasser,
(b) mit einer hydrophoben Phase enthaltend:
(b.1) eine oder mehrere Geminitensid-Verbindungen und
(b.2) ein oder mehrere Co-Amphiphile mit einem HLB- Wert von kleiner 6, im Gewichtsverhältnis Geminitensid-Verbindung (b.1) zu Co-Amphiphil (b.2) von 1 zu 100 bis 3 zu 1 Gewichtsteile,
(b.3) 1 bis 65 Gew.% einer hydrophoben Komponente,
und weiterhin enthaltend:
(c.1) Wasser, so dass eine Gesamtzusammensetzung enthaltend 15 Gew.% bis 45 Gew.% Wasser resultiert,
(c.2) Alkohole (c.2.1), Polyglykole (c.2.2) und/oder Polyole (c.2.3), so dass eine Gesamtzusammensetzung enthaltend in der Summe 0,1 bis 50 Gew.%, Alkohole (c.2.1), Polyglykole (c.2.2) und/oder Polyole (c.2.3) resultiert,
und eine oder mehrere der Komponenten (d.1) bis (d.3) durch Zugabe nach dem Zusammenbringen der Phasen (a) und (b), ggf. gemeinsam mit einer oder mehrer der Komponenten (c):
(d.1) 0,1 bis 30 Gew.% Feststoffpartikeln,
(d.2) 0,1 bis 3 Gew.% eines schäumenden Tensids oder
(d.3) 0,1 bis 15 Gew.% eines Antitranspirants,
wobei im Falle der Zugabe des schäumenden Tensids (d.2), eine Zugabe von zusätzlichem Wasser erfolgt, so dass sich ein Verhältnis von Öl in Wasser Emulsion ((a) bis (c)) zu zusätzlichem Wasser einschließlich schäumenden Tensid (d.2) von 90:10 bis 40 : 60 Gewichtsteilen ergibt und
wobei die Geminitenside mindestens zwei Tensideinheiten umfassen, die jeweils mindestens eine hydrophile Kopfgruppe und mindestens eine hydrophobe Gruppe aufweisen, und die Tensideinheiten durch mindestens einen Spacer in der Nähe der Kopfgruppe verknüpft sind.

2. O/W-Emulsion gemäß Anspruch 1 wobei Geminitensid-Verbindung (a.1) und Detergenskomponente (a.2) mit schlecht schäumender Charakteristik im Gewichtsverhältnis Geminitensid-Verbindung (a.1) zu Detergenskomponente (a.2) 1 zu 10 bis 4 zu 1 eingesetzt sind.

3. O/W-Emulsion gemäß einem der vorhergehenden Ansprüche, wobei die Komponenten (a.1) und (a.2) als Summe von Geminitensid-Verbindung (a.1) und Detergenskomponente (a.2) zu 0,05 Gew.% bis 5 Gew.% eingesetzt sind.

4. O/W-Emulsion gemäß einem der vorhergehenden Ansprüche, wobei Wasser (a.3) zu 2 bis 10 Gew.% zugesetzt ist.

5. O/W-Emulsion gemäß Anspruch 1 wobei Geminitensid-Verbindung (b.1) und Co-Amphiphil (b.2) im Gewichtsverhältnis Geminitensid-Verbindung (b.1) zu Co-Amphiphil (b.2) 1 zu 20 bis 1 zu 2 eingesetzt sind.

6. O/W-Emulsion gemäß einem der vorhergehenden Ansprüche, wobei die Komponenten (b.1) und (b.2) als Summe von Geminitensid-Verbindung (b.1) und Co-Amphiphil (b.2) zu 0,1 bis 8,0 Gew.% eingesetzt sind.

7. O/W-Emulsion gemäß einem der vorhergehenden Ansprüche, wobei 10 bis 40 Gew.% als hydrophobe Komponente (b.3) zugesetzt sind.

8. O/W-Emulsion gemäß einem der vorhergehenden Ansprüche, wobei der Phase (b) zusätzlich 0,01 bis 10 Gew.%, vorzugsweise 0,5 bis 3 Gew.%, eines oder mehrerer nichtionischer Teniside (b.4) zugesetzt sind.

9. O/W-Emulsion gemäß einem der vorhergehenden Ansprüche, wobei die O/W-Emulsion 15 Gew.% bis 45 Gew.% Wasser enthält, vorzugsweise durch Zugabe zur Phase (a) oder nach dem Zusammenbringen der Phasen (a) und (b).

10. O/W-Emulsion gemäß einem der vorhergehenden Ansprüche, wobei die O/W-Emulsion in der Summe 0,1 bis 50 Gew.%, vorzugsweise 5 bis 30 Gew.%, Alkohole (c.2.1), Polyglykole (c.2.2) und/oder Polyole (c.2.3) enthält.

11. O/W-Emulsion gemäß einem der vorhergehenden Ansprüche, wobei der Phase (a) vorzugsweise 0,1 bis 10 Gew.% Polyole (c.2.3) zugegeben sind.

12. O/W-Emulsion gemäß einem der vorhergehenden Ansprüche, wobei die O/W-Emulsion 0,01 bis 3 Gew.% eines Viskositätsregulators (c.4) enthält, vorzugsweise durch Zugabe zur Phase (a) oder nach dem Zusammenbringen der Phasen (a) und (b).

13. O/W-Emulsion gemäß einem der vorhergehenden Ansprüche, wobei die O/W-Emulsion 0,1 bis 1 Gew. %, vernetzte Polymere (c.5) enthält, vorzugsweise durch Zugabe nach dem Zusammenbringen der Phasen (a) und (b).

14. O/W-Emulsion gemäß einem der vorhergehenden Ansprüche, wobei die O/W-Emulsion 1 bis 8 Gew.% Feststoffpartikel (d.1) enthält.

15. O/W-Emulsion gemäß einem der vorhergehenden Ansprüche, wobei die O/W-Emulsion 0,5 bis 2 Gew.% eines schäumenden Tensids (d.2) enthält.

16. O/W-Emulsion gemäß einem der vorhergehenden Ansprüche, wobei die O/W-Emulsion als Antitranspirant (d.3) ein Metall-Chloro-Hydrats, vorzugsweise zu 2 bis 8 Gew. % enthält.

17. O/W-Emulsion gemäß einem der vorhergehenden Ansprüche, wobei die Komponenten (d.1), (d.2) oder (d.3) nach dem Zusammenbringen der Phasen (a) und (b), ggf. gemeinsam mit einer oder mehreren der Komponenten (c) zugegeben sind.

18. O/W-Emulsion gemäß einem der vorhergehenden Ansprüche, wobei die O/W-Emulsion 0,1 bis 20 Gew.%, vorzugsweise 1 bis 8 Gew.%, Ethanol enthält, vorzugsweise durch Zugabe zur Phase (a).

19. O/W-Emulsion gemäß einem der vorhergehenden Ansprüche, wobei die O/W-Emulsion 0,1 % bis 25 Gew.% eines öllöslichen Lichtschutzfilters (d.4) durch Zugabe zur Phase (b) oder 0,1 % bis 25 Gew.% eines wasserlöslichen Lichtschutzfilters oder seines Salzes (d.4) durch Zugabe zum nach dem Zusammenbringen der Phasen (a) und (b) zugesetzten Wasser, enthält.

20. O/W-Emulsion gemäß einem der vorhergehenden Ansprüche, wobei die O/W-Emulsion als Co-Amphiphil (b.2)
- ein C6- bis C40-, bevorzugt C8- bis C24-, Alkohol,
- eine C6- bis C24-, bevorzugt C8- bis C22-, Carbonsäure,
- ein Sorbitan(C6- bis C22-)ester ,
- ein Methylglucosid(C6- bis C22-)ester,
- ein Zucker(C6- bis C22-)ester,
- ein Mono-, Di- und Triglycerid einer C6- bis C22- Carbonsäure,
- ein mit Milchsäure oder Zitronensäure verestertes Derivat der Mono- und Diglyceride einer C6- bis C22-Carbonsäure,
- ein Polyglycerin(C6- bis C22-)ester,
- ein Propylenglycol(C6- bis C22-)ester,
- ein Vitaminester,
- Salicylsäure,
- Benzoesäure und / oder
- Lecithin
enthält bzw. das Co-Amphiphil (b.2) hieraus besteht.

21. O/W-Emulsion gemäß einem der vorhergehenden Ansprüche, wobei die O/W-Emulsion als Detergens-Komponente (a.2) Sulfosuccinate, Acyllactylate, Alkylpolyglucoside, Alkylisethionate, acylierte Proteinkondensate, Betaine und/oder Acylglutamate bzw. deren Derivate enthält bzw. die Detergens-Komponente (a.2) hieraus besteht.

22. O/W-Emulsion gemäß einem der vorhergehenden Ansprüche, wobei die O/W-Emulsion als Detergens-Komponente (a.2) Natrium-, Kalium-, Magnesium- oder Calciumsalze der an der Hydroxylgruppe mit linearen oder verzweigten, gesättigten oder 1 bis 3-fach nicht-benachbart ungesättigten, cyclischen oder acyclischen C₆- bis C₂₄- Carbonsäuren veresterten monomeren Milchsäure oder deren Oligomeren, wobei der Oligomerisierungsgrad der Milchsäure bei 1,1 bis 10, bevorzugt 1,1 bis 4 liegt, enthält bzw. die Detergens-Komponente (a.2) hieraus besteht.

23. Verfahren zur Herstellung der O/W-Emulsion gemäß einem der vorhergehenden Ansprüche, wobei die Zugabe der Phasen (a) und (b) bei 50 bis 80°C, vorzugsweise 60 bis 70°C, unter Homogenisieren durch Vermischen bis zu einer mittleren Teilchengröße der Öltropfchen gemäß Streulichtbestimmung von < 1 mm erfolgt.

## Claims

1. An oil-in-water emulsion (O/W emulsion) having a viscosity of less than 2,000 mPas, measured at 298 K, which is preparable by combining
(a) a hydrophilic phase containing
(a.1) one or more gemini surfactant compound(s) and
(a.2) a detergent component with poor foaming characteristic, at a ratio of gemini surfactant compound (a.1) to detergent component (a.2) from 1:100 to 10:1 and
(a.3) 1 to 15 wt% water,
(b) with a hydrophobic phase containing
(b.1) one or more gemini surfactant compound(s) and
(b.2) one or more co-amphiphile(s) having an HLB value of less than 6, at a weight ratio of gemini surfactant compound (b.1) to co-amphiphile (b.2) from 1:100 to 3:1 parts by weight,
(b.3) 1 to 65 wt% of a hydrophobic component,
and furthermore comprising
(c.1) water in such quantity that the resultant whole composition comprises 15 to 45 wt% water,
(c.2) alcohols (c.2.1), polyglycols (c.2.2) and/or polyols (c.2.3) such that the whole composition comprises a total of 0.1 to 50 wt% alcohols (c.2.1), polyglycols (c.2.2) and/or polyols (c.2.3),
and one or more of the components (d.1) through (d.3) by addition after combining the phases (a) and (b), optionally in conjunction with one or more of the components (c):
(d. 1) 0.1 to 30 wt% solid particles,
(d.2) 0.1 to 3 wt% of a foaming surfactant or
(d.3) 0.1 to 15 wt% of a antitranspirant,
with the proviso that when adding the foaming surfactant (d.2), additional water be added such that the proportion of the O/W emulsion comprised of components (a) to (c) to additional water, including the foaming surfactant (d.2), is from 90:10 to 40:60 parts by weight and
wherein the gemini surfactants comprise at least two surfactant units, having each at least one hydrophilic head group and at least one hydrophobic group, and the surfactant units are interlinked through at least one spacer near to the head group.

2. O/W emulsion of claim 1, wherein the gemini surfactant compound (a.1) and the detergent component (a.2) with poor foaming characteristics are employed at a weight ratio of gemini surfactant compound (a.1) to detergent component (a.2) from 1:10 to 4:1.

3. O/W emulsion according to any one of the preceding claims, wherein the components (a.1) and (a.2) as total of gemini surfactant compound (a.1) and detergent component (a.2) are employed of from 0.05 wt% to 5 wt%.

4. O/W emulsion according to any one of the preceding claims, wherein water (a.3) is added of from 2 to 10 wt%.

5. O/W emulsion of claim 1, wherein the gemini surfactant compound (b.1) and the co-amphiphile (b.2) are employed at a weight ratio of gemini surfactant compound (b.1) to co-amphiphile (b.2) of from 1:20 to 1:2.

6. O/W emulsion according to any one of the preceding claims, wherein the components (b.1) and (b.2) as a total of gemini surfactant compound (b.1) and co-amphiphile (b.2) are employed in quantities of from 0.1 to 8.0 wt%.

7. O/W emulsion according to any one of the preceding claims, wherein 10 to 40 wt% of the hydrophobic component (b.3) is added.

8. O/W emulsion according to any one of the preceding claims, wherein 0.01 to 10 wt% of one or more nonionic surfactant(s) (b.4), preferably 0.5 to 3 wt% is(are) additionally added to phase (b).

9. O/W emulsion according to any one of the preceding claims, wherein the O/W emulsion comprises 15 to 45 wt% water which is preferably added to phase (a) or after combining the phases (a) and (b).

10. O/W emulsion according to any one of the preceding claims, wherein the O/W emulsion comprises a total of 0.1 to 50 wt% alcohols (c.2.1), polyglycols (c.2.2) and/or polyols (c.2.3), preferably 5 to 30 wt%.

11. O/W emulsion according to any one of the preceding claims, wherein preferably 0.1 to 10 wt% polyols (c.2.3) are added to phase (a).

12. O/W emulsion according to any one of the preceding claims, wherein the O/W emulsion comprises 0.01 to 3 wt% of a viscosity regulator (c.4), preferably by addition to phase (a) or after combining the phases (a) and (b).

13. O/W emulsion according to any one of the preceding claims, wherein the O/W emulsion comprises 0.1 to 1 wt% cross-linked polymers (c.5), preferably added after combining the phases (a) and (b).

14. O/W emulsion according to any one of the preceding claims, wherein the O/W emulsion contains 1 to 8 wt% solid particles (d.1).

15. O/W emulsion according to any one of the preceding claims, wherein the O/W emulsion contains 0.5 to 2 wt% of a foaming surfactant (d.2).

16. O/W emulsion according to any one of the preceding claims, wherein the O/W emulsion comprises a metal chlorohydrate as antitranspirant (d.3), preferably of from 2 to 8 wt%.

17. O/W emulsion according to any one of the preceding claims, wherein the components (d.1), (d.2) or (d.3) are added after combining the phases (a) and (b), optionally in conjunction with one or more of components (c).

18. O/W emulsion according to any one of the preceding claims, wherein the O/W emulsion contains 0.1 to 20 wt%, preferably 1 to 8 wt%, ethanol preferably by addition to phase (a).

19. O/W emulsion according to any one of the preceding claims, wherein the O/W emulsion comprises 0.1 % to 25 wt% of a oil-soluble sun protection filter (d.4) added to phase (b) or 0.1 % to 25 wt% of a water-soluble sun protection filter or the salt thereof (d.4) added to the water which is added after combining the phases (a) and (b).

20. O/W emulsion according to any one of the preceding claims, wherein the O/W emulsion as a co-amphiphile (b.2) contains
- a C₆ to C₄₀ alcohol, preferably a C₈ to C₂₄ alcohol,
- a C₆ to C₂₄ carboxylic acid, preferably a C₈ to C₂₂ carboxylic acid,
- a sorbitan(C₆- to C₂₂-)ester,
- a methylglucoside(C₆- to C₂₂-)ester,
- a sugar(C₆- to C₂₂-)ester,
- a mono-, di-, and triglyceride of a C₆- to C₂₂- carboxylic acid,
- a derivative esterified with lactic acid or citric acid of the mono- and diglycerides of a C₆- to C₂₂-carboxylic acid,
- a polyglycerol(C₆- to C₂₂-)ester,
- a propyleneglycol(C₆- to C₂₂-)ester,
- a vitamin ester,
- salicylic acid,
- benzoic acid and/or
- lecithin,
or the co-amphiphile consist therefrom.

21. O/W emulsion according to any one of the preceding claims, wherein the O/W emulsion contains as detergent component (a.2) sulphosuccinates, acyllactylates, alkylpolyglucosides, alkylisethionates, acylated protein condensates, betaines and/or acylglutamates or the derivatives thereof, or the detergent component (a.2) consists therefrom.

22. O/W emulsion according to any one of the preceding claims, wherein the O/W emulsion as detergent component (a.2) sodium-, potassium-, magnesium- or calcium salts of monomeric lactic acid esterified on the hydroxyl group with linear or branched, saturated or mono- to tri- non-adjacent unsaturated, cyclic or acyclic C₆- to C₂₄-carboxylic acids, or the oligomers thereof, wherein the oligomerisation degree of the lactic acid is 1.1 to 10, preferably 1.1 to 4, or the detergent component (a.2) consists therefrom.

23. Process for preparing an O/W emulsion as claimed in any one of the preceding claims, wherein the phases (a) and (b) are added at 50 to 80 °C, preferably 60 to 70 °C, with homogenisation by mixing until the average particle size of the oil droplets is < 1 mm as determined by light scattering determination.

## Revendications

1. Emulsion huile-dans-eau (émulsion H/E) ayant une viscosité de moins de 2000 mPas, mesurée à 298 K, pouvant être préparée en réunissant :
(a) une phase hydrophile contenant :
(a.1) un ou plusieurs composés tensioactifs géminés et
(a.2) un composant détergent ayant une caractéristique de moussage médiocre, en un rapport entre le composé tensioactif géminé (a.1) et le composant détergent (a.2) de 1/100 à 10/1 et
(a.3) 1 à 15 % en poids d'eau,
(b) avec une phase hydrophobe contenant :
(b.1) un ou plusieurs composés tensioactifs géminés et
(b.2) un ou plusieurs co-amphiphiles ayant une valeur HLB de moins de 6, en un rapport en poids entre le composé tensioactif géminé (b.1) et le co-amphiphile (b.2) de 1/100 à 3/1 parties en poids, et
(b.3) 1 à 65 % en poids d'un composant hydrophobe,
et contenant également :
(c.1) de l'eau pour qu'il en résulte une composition totale contenant 15 % en poids à 45 % en poids d'eau,
(c.2) des alcools (c.2.1), des polyglycols (c.2.2) et/ou des polyols (c.2.3) pour qu'il en résulte une composition totale contenant au total 0,1 à 50 % en poids d'alcools (c.2.1), de polyglycols (c.2.2) et/ou de polyols (c.2.3),
et un ou plusieurs des composants (d.1) à (d.3), par addition, après la réunion des phases (a) et (b), le cas échéant conjointement avec un ou plusieurs des composants (c) :
(d.1) 0,1 à 30 % en poids de particules solides,
(d.2) 0,1 à 3 % en poids d'un tensioactif moussant ou
(d.3) 0,1 à 15 % en poids d'un antitranspirant,
où, dans le cas de l'addition du tensioactif moussant (d.2), une addition d'eau supplémentaire s'effectue si bien qu'il en résulte un rapport entre l'émulsion huile-dans-eau ((a) à (c)) et l'eau supplémentaire, tensioactif moussant (d.2) inclus, de 90/10 à 40/60 parties en poids et
où les tensioactifs géminés comprennent au moins deux motifs tensioactifs, qui présentent respectivement au moins un groupe de tête hydrophile et au moins un groupe hydrophobe, et les motifs tensioactifs sont liés par au moins un écarteur à proximité du groupe de tête.

2. Emulsion H/E selon la revendication 1, où le composé de tensioactif géminé (a.1) et le composant détergent (a.2) ayant une caractéristique de moussage médiocre sont mis en oeuvre en un rapport en poids entre le composé de tensioactif géminé (a.1) et le composant détergent (a.2.) de 1/10 à 4/1.

3. Emulsion H/E selon l'une des revendications précédentes, où les composants (a.1) et (a.2) sont mis en oeuvre en tant que somme du composé tensioactif géminé (a.1) et du composant détergent (a.2) en une quantité de 0,05 % en poids à 5 % en poids.

4. Emulsion H/E selon l'une des revendications précédentes, où l'eau (a.3) est additionnée en une quantité de 2 à 10 % en poids.

5. Emulsion H/E selon la revendication 1, où le composé tensioactif géminé (b.1) et le co-amphiphile (b.2) sont mis en oeuvre en un rapport en poids entre le composé tensioactif géminé (b.1) et le co-amphiphile (b.2) de 1/20 à ½.

6. Emulsion H/E selon l'une des revendications précédentes, où les composants (b.1) et (b.2) sont mis en oeuvre en tant que somme du composé tensioactif géminé (b.1) et du co-amphiphile (b.2) en une quantité de 0,1 % en poids à 8,0 % en poids.

7. Emulsion H/E selon l'une des revendications précédentes, où l'on ajoute 10 à 40 % en poids du composant hydrophobe (b.3).

8. Emulsion H/E selon l'une des revendications précédentes, où la phase (b) est additionnée en plus de 0,01 à 10 % en poids, de préférence de 0,5 à 3 % en poids d'un ou de plusieurs tensioactifs non ioniques (b.4).

9. Emulsion H/E selon l'une des revendications précédentes, où l'émulsion H/E comprend 15 % en poids à 45 % en poids d'eau, de préférence par addition à la phase (a) ou après la réunion des phases (a) et (b).

10. Emulsion H/E selon l'une des revendications précédentes, où l'émulsion H/E comprend au total 0,1 à 50 % en poids, de préférence 5 à 30 % en poids d'alcools (c.2.1), de polyglycols (c.2.2) et/ou de polyols (c.2.3).

11. Emulsion H/E selon l'une des revendications précédentes, où la phase (a) est additionnée de préférence de 0,1 à 10 % en poids de polyols (c.2.3).

12. Emulsion H/E selon l'une des revendications précédentes, où l'émulsion H/E comprend 0,01 à 3 % en poids d'un régulateur de viscosité (c.4), de préférence par addition à la phase (a) ou après la réunion des phases (a) et (b).

13. Emulsion H/E selon l'une des revendications précédentes, où l'émulsion H/E comprend 0,1 à 1 % en poids de polymères réticulés (c.5), de préférence par addition après la réunion des phases (a) et (b).

14. Emulsion H/E selon l'une des revendications précédentes, où l'émulsion H/E comprend 1 à 8 % en poids de particules solides (d.1).

15. Emulsion H/E selon l'une des revendications précédentes, où l'émulsion H/E comprend 0,5 à 2 % en poids d'un tensioactif moussant (d.2).

16. Emulsion H/E selon l'une des revendications précédentes, où l'émulsion H/E comprend à titre d'antitranspirant (d.3) un chlorhydrate de métal, de préférence en une quantité de 2 à 8 % en poids.

17. Emulsion H/E selon l'une des revendications précédentes, où les composants (d.1), (d.2) ou (d.3) sont ajoutés après la réunion des phases (a) et (b), le cas échéant conjointement avec un ou plusieurs des composants (c).

18. Emulsion H/E selon l'une des revendications précédentes, où l'émulsion H/E comprend 0,1 à 20 % en poids, de préférence 1 à 8 % en poids d'éthanol, de préférence par addition à la phase (a).

19. Emulsion H/E selon l'une des revendications précédentes, où l'émulsion H/E comprend 0,1 % à 25 % en poids d'un filtre photoprotecteur liposoluble (d.4) par addition à la phase (b) ou 0,1 % à 25 % en poids d'un filtre photoprotecteur hydrosoluble ou de son sel (d.4) par addition à l'eau ajoutée après la réunion des phases (a) et (b).

20. Emulsion H/E selon l'une des revendications précédentes, où l'émulsion H/E comprend à titre de co-amphiphile (b.2)
- un alcool en C₆ à C₄₀, de préférence en C₈ à C₂₄,
- un acide carboxylique en C₆ à C₂₄, de préférence en C₈ à C₂₂,
- un ester (en C₆ à C₂₂) de sorbitane,
- un ester (en C₆ à C₂₂) de méthylglucoside,
- un ester (en C₆ à C₂₂) de sucre,
- un mono-, di- et triglycéride d'un acide carboxylique en C₆ à C₂₂,
- un dérivé estérifié avec de l'acide lactique ou de l'acide citrique des mono- et diglycérides d'un acide carboxylique en C₆ à C₂₂,
- un ester (en C₆ à C₂₂) de polyglycérine,
- un ester (en C₆ à C₂₂) de propylène glycol,
- un ester de vitamine,
- l'acide salicylique,
- l'acide benzoïque et/ou
- la lécithine
ou bien le co-amphiphile (b.2.) s'en compose.

21. Emulsion H/E selon l'une des revendications précédentes, où l'émulsion H/E comprend à titre de composant détergent (a.2) des sulfosuccinates, des acyllactates, des alkylpolyglucosides, des alkyliséthionates, des condensés de protéines acylés, des bétaïnes et/ou des acylglutamates ou leurs dérivés ou bien le composant détergent (a.2) s'en compose.

22. Emulsion H/E selon l'une des revendications précédentes, où l'émulsion H/E comprend à titre de composant détergent (a.2) des sels de sodium, de potassium, de magnésium ou de calcium de l'acide lactique monomère, estérifié sur le groupe hydroxyle par des acides carboxyliques en C₆ à C₂₄ cycliques ou acycliques, saturés ou 1 à 3 fois insaturés de manière non adjacente, linéaires ou ramifiés, ou de ses oligomères, le degré d'oligomérisation de l'acide lactique se situant vers 1,1 à 10, de préférence de 1,1 à 4, ou bien le composant détergent (a.2) s'en compose.

23. Procédé de préparation de l'émulsion H/E selon l'une des revendications précédentes, où l'addition des phases (a) et (b) à une température de 50 à 80°C, de préférence de 60 à 70°C, s'effectue sous homogénéisation par mélange jusqu'à une granulométrie moyenne des gouttelettes d'huile, selon la détermination par lumière dispersée, de < 1 mm.
